# EUROPEAN PATENT SPECIFICATION

(11) **EP 3 286 565 B1**
(45) Date of publication and mention of the grant of the patent: **15.01.2025**
(21) Application number: 16722737.0
(22) Date of filing: 20.04.2016
(51) Int. Cl.: C07K 16/30, C07K 16/40, A61K 47/68, G01N 33/574

(54) **COMPOSITIONS AND METHODS FOR PROSTATE CANCER ANALYSIS**
ZUSAMMENSETZUNGEN UND VERFAHREN FÜR PROSTATAKREBSANALYSE
COMPOSITIONS ET PROCÉDÉS POUR L'ANALYSE DU CANCER DE LA PROSTATE

(30) Priority: 21.04.2015 US 201562150724 P
(43) Date of publication of application: 28.02.2018
(73) Proprietor: Genentech, Inc., South San Francisco, CA 94080 (US); Epic Sciences Inc., San Diego, CA 92121 (US)
(72) Inventor: KABBARAH, Omar, South San Francisco, California 94080 (US); SZAFER-GLUSMAN, Iliana, San Jose, California 95124 (US); MARRINUCCI, Dena, San Diego, California 92121 (US); LEE, Florence, San Diego, California 92121 (US); WERNER, Shannon, San Diego, California 92121 (US)
(74) Representative: Mewburn Ellis LLP
(86) International application number: PCT/US2016/028372
(87) International publication number: WO 2016/172160

(56) References cited:
- WO-A1-2015/161231
- US-A1- 2013 143 237
- WANG G ET AL: "Tyramide Signal Amplification Method in Multiple-Label Immunofluorescence Confocal Microscopy", METHODS, ACADEMIC PRESS, US, vol. 18, no. 4, 1 August 1999 (1999-08-01), pages 459 - 464, XP004466790, ISSN: 1046-2023, DOI: 10.1006/METH.1999.0813

## Description

### RELATED APPLICATIONS

This application claims the benefit of priority of provisional U.S. Application No. 62/150,724 file April 21, 2015.

### FIELD OF THE INVENTION

The present invention relates to the field of oncology and cancer diagnosis, and more specifically to compositions and methods for prostate cancer screening, staging, and treatment monitoring. In specific embodiments, the present invention relates to novel prognostic and diagnostic tests for prostate cancer.

### BACKGROUND

Prostate cancer is one of the most prevalent cancers in men. While most prostate cancers at early onset are symptom-free and slow growing, certain prostate cancers are more aggressive, painful and lead to fatality. At present, two major types of non-invasive screening tests are available for detection of prostate cancer in men. One is digital rectal exam (DRE), which allows a doctor to detect prostate abnormalities by inserting a gloved finger into the rectum and feeling the prostate gland, and the other is prostate surface antigen test (PSA test), which measures the level of the PSA antigen in a blood sample. Although FDA has approved use of PSA test together with DRE to help detect prostate cancer in men, the PSA test is controversial in screening as it is not clear whether the test actually saves lives. In particular, the United States Preventive Services Task Forces has recently recommended against PSA screening in healthy men, based on findings that PSA screening reduces no or little prostate cancer mortality while leading to treatments or tests that cause unnecessary pain and side effects (*see, e.g.,* R. Chou et al, Ann. Intern. Med., October 7, 2011 E-375; Djulbegovic et al, BMJ 2010, 341: c4543). The most definitive diagnosis of prostate cancer is biopsy, where a small piece of the prostate from the suspected patient is removed for microscopic examination for the presence of tumor cells. Obviously such procedure is rather invasive and less desirable in early screening and detection. Accordingly, in view of the recent finding that PSA screening fails in reducing mortality and biopsy is invasive, there remain significant needs for development of agents and methods that can provide reliable results in the diagnosis and prognosis of prostate cancer.

As in many other types of cancers, the main cause of death in prostate cancer patients is not the primary tumor but rather the metastasis. Some primary tumor cells can detach themselves from the original tissue and enter into circulation. These cells are called circulating tumor cells (CTCs). Once CTCs seed themselves to a suitable site in the body, they may develop into metastatic colonies that are difficult to detect yet can be life-threatening as they progress into secondary tumors.

Detection and enumeration of CTCs is important for patient care for a number of reasons. They may be detectable before the primary tumor, thus allowing early stage diagnosis. They decrease in response to therapy, so the ability to enumerate CTCs allows one to monitor the effectiveness of a give therapeutic regimen. Furthermore, CTC diagnostics can be used as a tool to monitor for recurrence in patients with no measurable disease in the adjuvant setting. For example, CTC were found to be present in 36% of breast cancer patients 8-22 years after mastectomy, apparently from micrometastases (deposits of single tumor cells or very small clusters of neoplastic cells). Meng et al., Clin. Can. Res. 1024): 8152-62, 2004. However, while CTC diagnostics offers the promise of detecting cancer where traditional diagnostic tests fail, current methods are limited by their sensitivity to detect specific tumor markers (*e.g.,* STEAP1).

In addition, CTCs may be used to predict progression-free survival (PFS) and overall survival (OS), as the presence/number of circulating tumor cells in patients with metastatic carcinoma has been shown to be correlated with both PFS and OS. See e.g., Cristofanilli et al., J. Clin. Oncol. 23(1): 1420-1430, 2005; Cristofanilli et al., N. Engi. J. Med. 351(8): 781-791, 2004. However, just as there is a need to enhance the sensitivity CTC diagnostics, there is also a need to enhance the sensitivity of CTC tests that detect specific tumor markers (*e.g.,* STEAP1) to determine PFS. For example, US 2013/143237 A1 involves a method of detecting, grading and monitoring prostate CTCs using anti-STEAP1 antibodies and DAPI immunofluorescence. The methods disclosed uses the CellSearch^{®} system for initial identification and enrichment of epithelial cells followed by immunofluorescence detection.

There are multiple approved treatment options for prostate cancer and many others in clinical trials. Treatment options include, but are not limited to, active surveillance and monitoring without giving any treatment until symptoms appear and/or change, surgery (e.g., retropubic prostatectomy, perineal prostatectomy, pelvic lymphadenectomy, and transurethral resection of the prostate), radiation therapy (e.g., external radiation therapy, progression internal radiation therapy, and/or alpha emitter radiation therapy), hormone therapy (e.g., luteinizing hormone-releasing agonists, antiandrogens such as enzalutamide, drugs that prevent the adrenal glands from making androgens, an orchiectomy, and estrogens), chemotherapy (e.g., abiraterone, bicalutamide, cabazitaxel, casodex, degarelix, docetaxel, enzalutamide, goserelin acetate, leuprolide acetate, mitoxantrone hydrochloride, prednisone, sipulcucel-T, and radium 223 dichloride), biologic therapy, and bisphosphonate therapy.

Specifically with regard to biologic therapy, antibody drug conjugates (ADCs) are of particular interest because they hold promise for enhancing the therapeutic index of cytotoxics. STEAP1 is overexpressed in metastatic castration resistant prostate cancer (mCRPC) and is the target of an ADC in clinical development. However, a companion diagnostic is needed to identify patients who will respond to a STEAP1-ADC therapy. Similarly, a companion diagnostic is needed to identify patients who are responding to STEAP1-ADC therapy. Accordingly, there is an ongoing need for companion diagnostics to predict the efficacy of ADC therapies in order to optimize and develop new patient treatment.

To identify patients most likely to benefit from the STEAP1-ADC therapy, STEAP1 expression was used as a predictive biomarker in archival and/or newly acquired tumor tissue using a CLIA-certified IHC assay, and on circulating tumor cells (CTCs) in blood using EPIC platforms. Specifically, novel multi-channel diagnostic and prognostic tests with enhanced sensitivity to identify STEAP1 positive CTCs were developed and disclosed herein to predict and assess efficacy of the STEAP1-ADC therapy. Accordingly, the benefit of the STEAP1-ADC CTC assays disclosed herein is 1) the new assays can detect patients with STEAP1+ mCRPC that old assays did not have the sensitivity to detect, 2) the new assays can detect STEAP 1+ mCRPC patients without invasive and painful biopsies that are candidates for the STEAP1-ADC therapy, 3) the new assays are the first to be able to monitor STEAP 1 expression throughout treatment without invasive and painful biopsies.

### SUMMARY

In one aspect, the present invention provides methods for diagnosing prostate cancer in a test subject using CTCs, comprising: a) identifying CTCs from a blood sample taken from the test subject wherein the identified CTCs are not separated or enriched from the other components in the blood sample; b) contacting the CTCs with an antibody that specifically binds to STEAP1, an antibody that specifically binds to CD45, and an antibody that specifically binds to cytokeratin; c) staining the CTCs with DAPI; and d) detecting the DAPI stain and bound antibodies; wherein the anti-STEAP1 bound antibody is detected with a tyramiude signal amplification (TSA) protocol to enhance signal; wherein the presence of CTCs that express cytokeratin and the prostate-specific marker STEAP1 and exhibit DAPI staining without CD45 expression is predictive of having prostate cancer in the test subject.

In specific embodiments, the anti-STEAP1 antibody is the 15A5 antibody, produced by a hybridoma cell having a microorganism deposit number of PTA-12259.

In specific embodiments, the methods further comprise determining the expression level of STEAP1 on the cancer cells.

In specific embodiments, the methods further comprise grading the cancer cells based on their expression level of STEAP1, and determining the percentage of the cancer cells in each grade. In specific embodiments, the methods further comprise calculating a grade score for each grade by multiplying the percentage of the cancer cells in said grade with a unique grade number representative of the expression level of the prostate-specific marker in said grade, and summing up all the grade scores to obtain an H score, wherein the H score is indicative of the stage of the prostate cancer in the test subject.

Disclosed herein are methods in which, the cancer cells are identified from the blood sample with a capturing composition comprising a ligand that specifically binds to cancer cells of epithelial origin. In some examples, the ligand is an antibody that specifically binds to an epithelial antigen preferentially expressed on cancer cells. In some examples, the epithelial antigen is Epithelial Cell Adhesion Molecule (EpCAM).

Disclosed herein are methods in which, the identified cancer cells are enriched in a cell fraction separated from the blood sample. In some examples, the cell fraction is separated under a magnetic field. In some examples, the ligand in the capturing composition is coupled to a magnetic particle (*e.g.,* colloidal magnetic particle, *e.g.,* colloidal magnetic nanoparticles). In some examples, the ligand comprises an EpCAM antibody.

In certain embodiments, the anti-STEAP-1 antibody binds to STEAP-1 with a K_{D} of ≤ 1000 nM.

In certain embodiments, the anti-STEAP-1 antibody, the anti-CD45 antibody, and/or the anti-cytokeratin antibody are conjugated with detectable labels.

In one aspect, the present invention provides methods of predicting efficacy of STEAP1-ADC therapy in a test subject suffering from prostate cancer, comprising: a) identifying CTCs from a blood sample taken from the test subject wherein the identified CTCs are not separated or enriched from the other components in the blood sample; b) contacting the CTCs with an antibody that specifically binds to STEAP1, an antibody that specifically binds to CD45, and an antibody that specifically binds to cytokeratin; c) staining the CTCs with DAPI; and d) detecting the DAPI stain and bound antibodies, wherein the anti-STEAP1 antibody is detected with a tyramide signal amplification (TSA) protocol to enhance signal, wherein the presence of CTCs that express cytokeratin and STEAP1 and exhibit DAPI staining without CD45 expression is predictive of efficacy of the STEAP1-ADC.

In one aspect, the present invention provides methods of monitoring response to STEAP1-ADC therapy in a test subject suffering from prostate cancer using a tyramide signal amplification (TSA) protocol to enhance signal, comprising: a) contacting a first group of CTCs with an antibody that specifically binds to STEAP1, an antibody that specifically binds CD45, and an antibody that specifically binds cytokeratin, and staining the cells with DAPI, wherein the first group of cancer cells are from a first blood sample taken from the test subject before treatment with the STEAP1-ADC; b) determining the number of CTCs in the first group that express STEAP1 and cytokeratin and stain with DAPI; c) contacting a second group of CTCs with an antibody that specifically binds to STEAP1, an antibody that specifically binds CD45, and an antibody that specifically binds cytokeratin, and staining the cells with DAPI, wherein the second group of cancer cells are from a second blood sample taken from the test subject during or after treatment with the STEAP1-ADC; d) determining the amount of the cancer cells in the second group that express STEAP1 and cytokeratin and stain with DAPI; and e) comparing the amount of the CTCs that express STEAP1 and cytokeratin and stain with DAPI as determined in b) with that in d), wherein a decrease in the amount of the CTCs and/or a decrease in the STEAP1+ CTCs indicates a response to the prostate cancer therapy in the test subject, wherein the anti-STEAP1 bound antibody is detected with a tyramide signal amplification (TSA) protocol to enhance signal; and wherein the CTCs are not separated or enriched from the other components in the blood sample.

In certain embodiments, the STEAP1-ADC comprises an anti-STEAP-1 antibody covalently attached to a cytotoxic agent. In specific embodiments, the cytotoxic agent is selected from a toxin, a chemotherapeutic agent, a drug moiety, monomethylauristatin E (MMAE), an antibiotic, a radioactive isotope, and a nucleolytic enzyme. In certain embodiments, the DAPI, STEAP1, CD45, and cytokeratin signals are detected using FACS. In other embodiments, the DAPI, STEAP1, CD45, and cytokeratin signals are detected using immunohistochemistry.

### BRIEF DESCRIPTION OF THE FIGURES

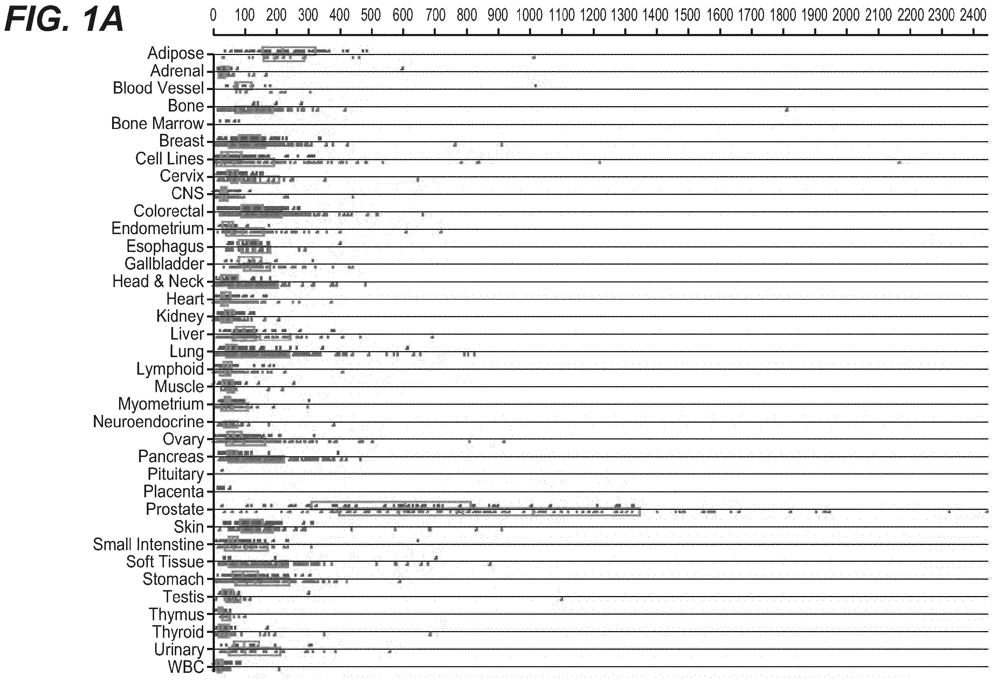

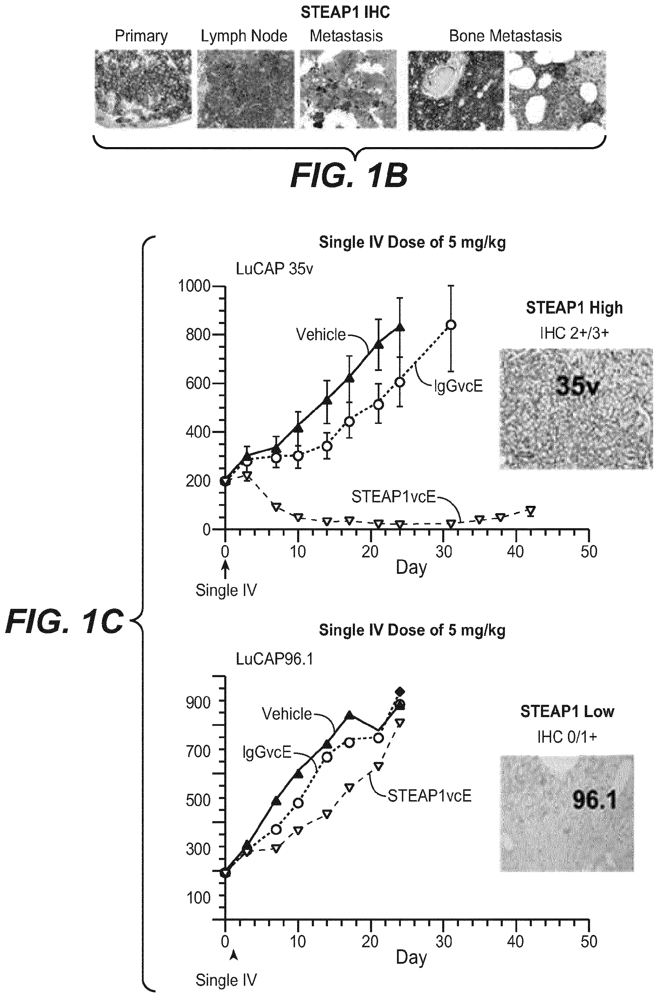

**Figure 1** shows that STEAP1 is highly expressed in normal prostate epithelium and the majority of primary prostate tumors. Fig. 1A shows STEAP1 Affymetrix signal across multiple tissues. Fig. 1B shows that STEAP1 expression is maintained in in prostate cancer metastases. 37/42 (88%) of primary prostatic adenocarcinomas were IHC STEAP1 positive and 24/28 (86%) of metastatic prostatic adenocarcinomas were IHC STEAP1 positive. Fig. 1C shows robust pre-clinical activity of the STEAP1-ADC.

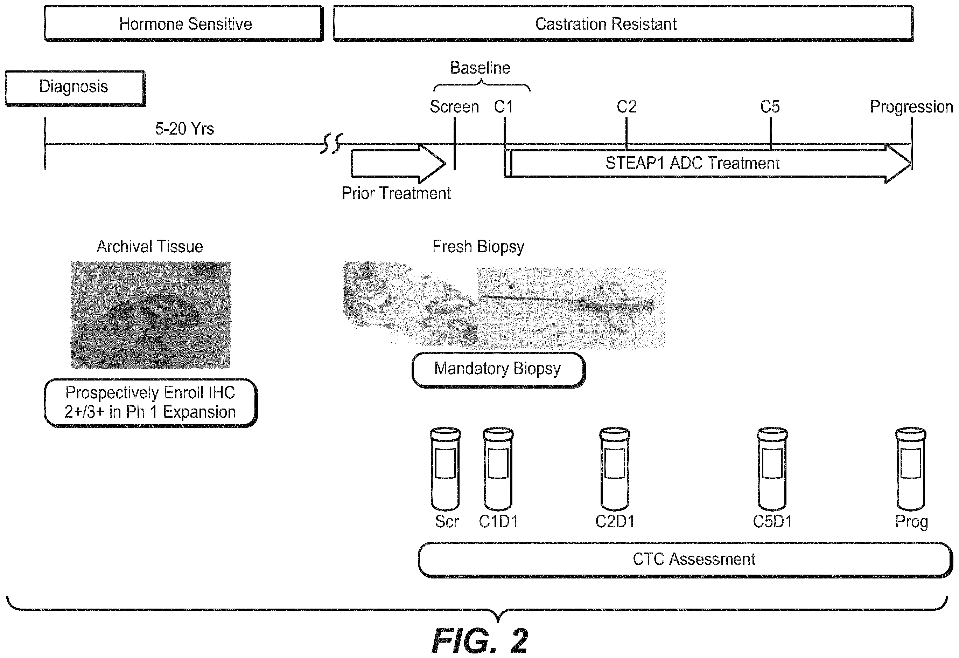

**Figure 2** shows a schematic of the bio-sample collection plan and methodologies for measuring STEAP1 levels in tissue and blood. Specifically, it shows the comprehensive tissue and blood collection plan over the course of disease history, treatment, and progression.

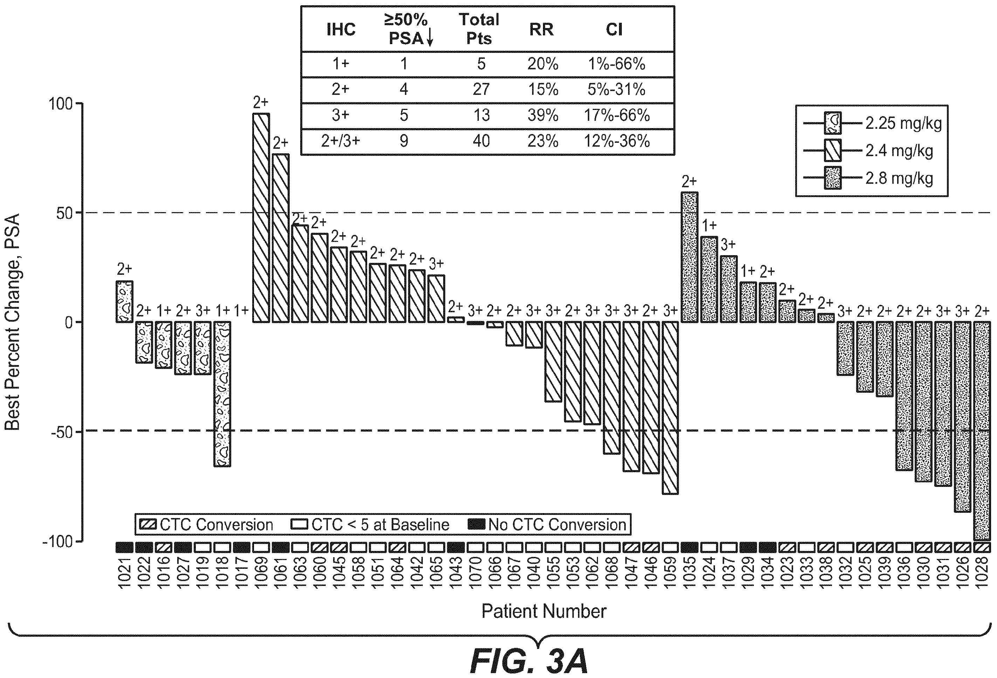

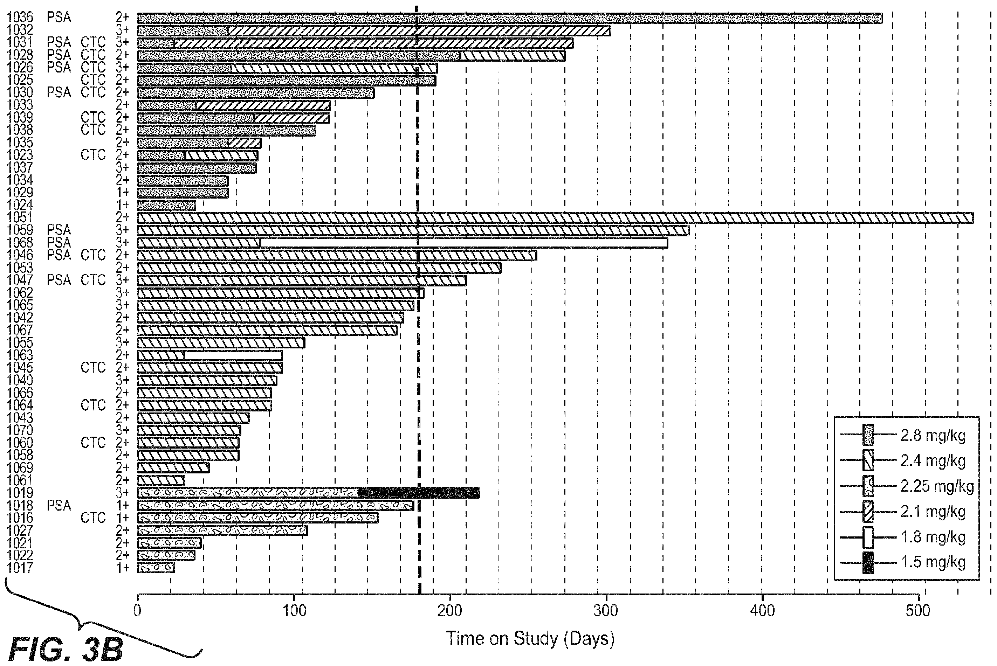

**Figure 3** shows that patients with STEAP1-high expressing tumors tend to exhibit the highest PSA declines and longest stays on study. Fig. 3A shows that PSA Responses of ≥50% compared to baseline levels were most frequently observed in patients with STEAP1 IHC 3+ tumors. Fig. 3B shows that times on study of >6months were most frequently observed for patients with STEAP1 IHC 3+ tumors.

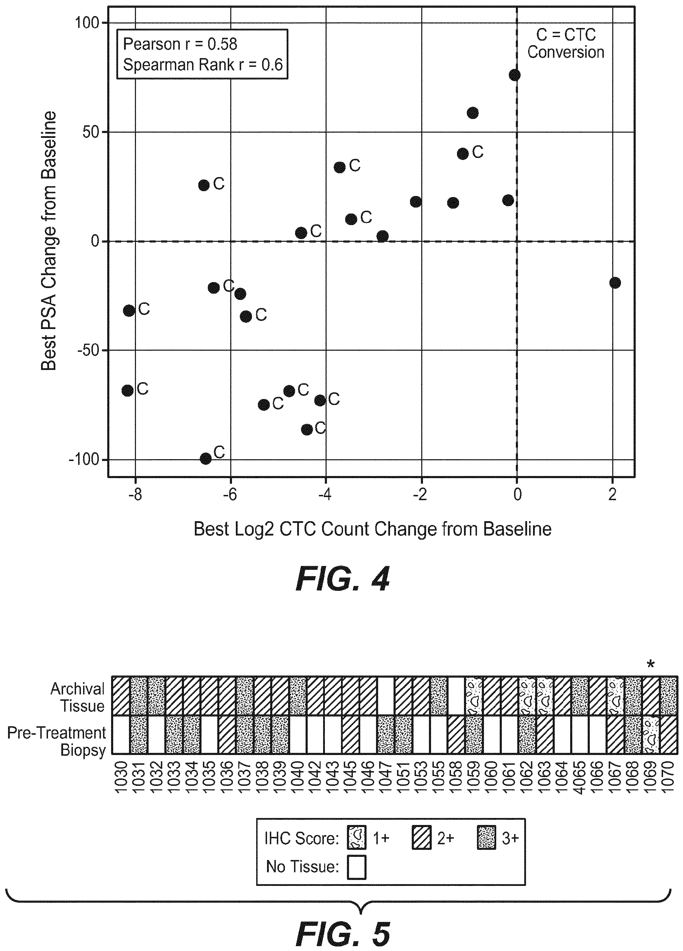

**Figure 4** shows the correlation between PSA reductions and CTC conversions. Best PSA reductions from baseline were observed in patients who exhibited CTC conversions from the prognostically unfavorable counts of ≥5CTCs/7.5mls to the favorable <5CTCs/7.5mls of blood.
**Figure 5** shows that STEAP1 expression is stable in archival and baseline tissues. STEAP1 levels as measured by IHC were either similar or higher in baseline biopsies compared to archival samples in all but one case.

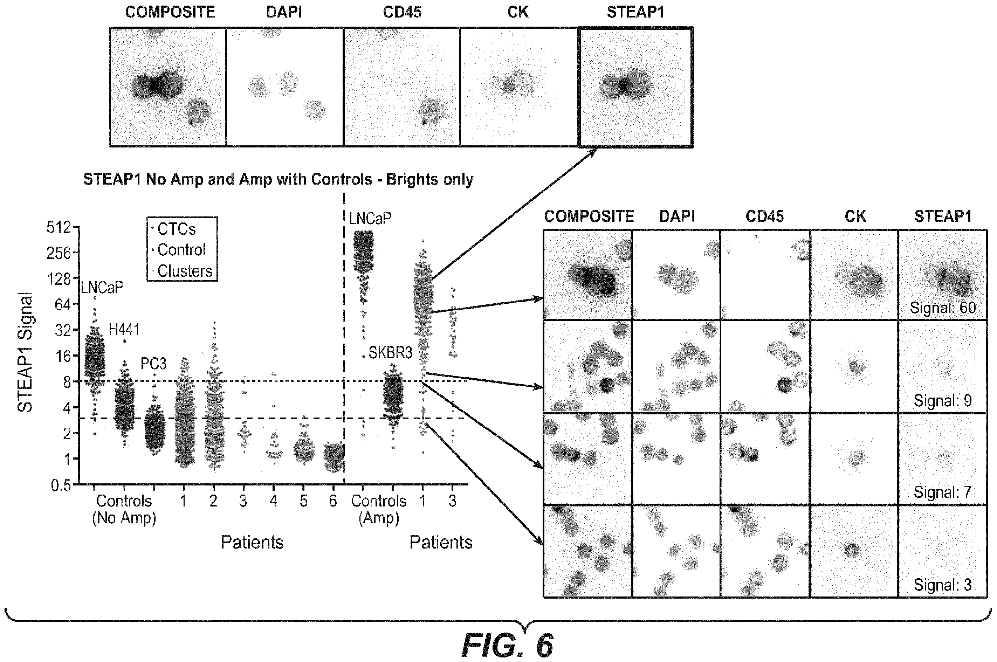

**Figure 6** shows that STEAP1-ADC treatment depletes STEAP1+ CTCs. Two novel 4-color IF-based assays were developed to measure the expression of STEAP 1 on the surface of CTCs using a traditional protocol (non-amp) and a novel signal amplification protocol (amp protocol).
**Figures 7A** **and &7B** show that a reduction in the total number of CTCs/ml as well as in the number of STEAP 1+ CTC was observed after treatment with the STEAP 1-ADC.
**Figures 8A** and **8B** show immunofluorescence-based detection of STEAP1 expression levels on CTCs.
**Figure 9** shows that the signal amplification protocol (the tyramide signal amplification (TSA) protocol) increases the dynamic range of the STEAP1 CTC assay in control cells. Fig. 9A shows that the STEAP1 signal increases by 8.1-fold with the amplification protocol while the background only increases by 2.4-fold. Fig. 9B shows that the amplification protocol is capable of identifying STEAP1+ patients who cannot be identified with the traditional non-amplification protocols.
**Figure 10** shows patient case studies where treatment with the STEAP1-ADC results in a decrease in both total CTCs/mL and STEAP+ CTC counts.

### DETAILED DESCRIPTION OF THE INVENTION

### I. DEFINITIONS

The term "tumor" or "cancer", as used interchangeably herein, refers to any medical condition characterized by neoplastic or malignant cell growth, proliferation, or metastasis, and includes both solid cancers and non-solid cancers such as leukemia.

The term "prostate cancer" or "prostate tumor" as used herein, refers to cancer or tumor that is originated from a prostate tissue.

The term "stage" in the context of a disease (such as cancer or tumor), refers to the progression status of the disease which is indicative of the severity of the disease.

The term "staging" as used herein refers to identifying the particular stage at which the disease has progressed.

The term "diagnosis" (along with grammatical variations thereof such as "diagnosing" or "diagnostic") refers to the identification of a molecular or pathological state, disease or condition, such as the identification of cancer, or refers to the identification of a cancer patient who may benefit from a particular treatment regimen.

The term "prognosis" (and grammatical variations thereof such as "prognosing" or "prognostic") refers to the prediction of the likelihood of benefit from a treatment such as a cancer therapy.

The term "prediction" or "predicting" is used herein to refer to the likelihood that a patient will respond either favorably or unfavorably to a particular anti-prostate cancer therapy. In one embodiment, prediction or predicting relates to the extent of those responses. In one embodiment, the prediction or predicting relates to whether and/or the probability that a patient will survive or improve following treatment, for example treatment with a particular therapeutic agent, and for a certain period of time without disease progression.

The term "benefit" is used in the broadest sense and refers to any desirable effect and specifically includes clinical benefit as defined herein.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.,* mice and rats). In certain embodiments, the individual or subject is a human.

The term Six-Transmembrane Epithelial Antigen of the Prostate 1, also called STEAP-1, refers to a cell surface antigen predominantly expressed in prostate tissue, and is found to be upregulated in multiple cancer cell lines. Hubert et al. (1999), Proc. Natl. Acad. Sci. USA, 96(25), 14523-8. An exemplary human STEAP-1 has an amino acid sequence of SEQ ID NO: 1 disclosed in US 2009/0280056 A1, filed 26 Oct 2007.

The terms "anti-STEAP-1 antibody" and "an antibody that binds to STEAP-1" refer to an antibody that is capable of binding STEAP-1 with sufficient affinity such that the antibody is useful as a diagnostic agent in targeting STEAP-1. In one embodiment, the extent of binding of an anti-STEAP-1 antibody to an unrelated, non-STEAP-1 protein is less than about 10% of the binding of the antibody to STEAP-1 as measured, *e.g.*, by a radioimmunoassay (RIA). In certain embodiments, an antibody that binds to STEAP-1 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g.* 10⁻⁸ M or less, *e.g.* from 10⁻⁸ M to 10⁻¹³ M, *e.g.,* from 10⁻⁹ M to 10⁻¹³ M). In certain embodiments, an anti-STEAP-1 antibody binds to an epitope of STEAP-1 that is conserved among STEAP-1 from different species.

The term "STEAP1-ADC" refers to an anti-STEAP1 antibody-drug conjugate.

The term "CTC" refers to circulating tumor cells. CTCs are cells of epithelial origin that are present in the circulation of patients with different solid malignancies. They are derived from clones of the primary tumor and are malignant. (*See* Fehm et al., Clin. Cancer Res. 8: 2073-84, 2002.) Specifically, CTCs shed into the vasculature from a primary tumor and circulate throughout the bloodstream and can serve as the "seeds" for subsequent metastases in vital distant organs, triggering a mechanism that is responsible for the vast majority of cancer-related deaths. Futhermore, Evidence has accumulated in the literature showing that CTCs can be considered an independent diagnostic for cancer progression of carcinomas (Beitsch & Clifford, Am. J. Surg. 180(6): 446-49, 2000 (breast); Feezor et al., Ann. Oncol. Surg. 9(10): 944-53, 2002 (colorectal); Ghossein et al., Diagn. Mol. Pathol. 8(4): 165-75, 1999 (melanoma, prostate, thyroid); Glaves, Br. J. Cancer 48: 665-73, 1983 (lung); Matsunami et al., Ann. Surg. Oncol. 10(2): 171-5, 2003 (gastric); Racila et al., 1998; Pantel et al., 1999).

"Affinity" refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g.,* antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

The term "antibody" herein is used in the broadest sense and encompasses various antibody structures, including but not limited to monoclonal antibodies, polyclonal antibodies, multispecific antibodies (*e.g.,* bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (*e.g.* scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "antibody 15A5" as used herein refers to a mouse monoclonal anti-STEAP-1 antibody produced by a hybridoma cell line having a microorganism deposit number of PTA-12259. The microorganism deposit information of the hybridoma cell line is as follows: ATCC Deposit No.: PTA-12259; Deposit Date: November 17, 2011; and Material Deposited: hybridoma 15A5.1.1.1 (also designated 7284), which produces antibody 15A5.

The "class" of an antibody refers to the type of constant domain or constant region possessed by its heavy chain. There are five major classes of antibodies: IgA, IgD, IgE, IgG, and IgM, and several of these may be further divided into subclasses (isotypes), *e.g.*, IgG₁, IgG₂, IgG₃, IgG₄, IgA₁, and IgA₂. The heavy chain constant domains that correspond to the different classes of immunoglobulins are called α, δ, ε, γ, and µ, respectively.

The terms "full length antibody," "intact antibody," and "whole antibody" are used herein interchangeably to refer to an antibody having a structure substantially similar to a native antibody structure or having heavy chains that contain an Fc region as defined herein.

The term "monoclonal antibody" as used herein refers to an antibody obtained from a population of substantially homogeneous antibodies, i.e., the individual antibodies comprising the population are identical and/or bind the same epitope, except for possible variant antibodies, *e.g.,* containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "cytotoxic agent" as used herein refers to a substance that inhibits or prevents a cellular function and/or causes cell death or destruction. Cytotoxic agents include, but are not limited to, radioactive isotopes (*e.g.*, At²¹¹, I¹³¹ , I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (*e.g.,* methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; and toxins such as small molecule toxins or enzymatically active toxins of bacterial, fungal, plant or animal origin, including fragments and/or variants thereof. Non-limiting examples of cytotoxic agents suitable for the present invention include those described in US 2009/0280056 A1, filed 26 Oct 2007. For example, in certain embodiments, a cytotoxic agent is monomethyl auristatin E (MMAE).

A "cysteine engineered antibody" or "cysteine engineered antibody variant" is an antibody in which one or more residues of an antibody are substituted with cysteine residues. The thiol group(s) of the cysteine engineered antibodies can be conjugated to a drug moiety (*e.g.,* via a linker) to form a THIOMAB^{™} antibody (*i.e.,* a THIOMAB^{™} drug conjugate (TDC)). In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. For example, a THIOMAB^{™} antibody may be an antibody with a single mutation of a non-cysteine native residue to a cysteine in the light chain (*e.g.,* G64C, K149C, R142C, and V205C according to Kabat numbering) or in the heavy chain (*e.g.,* D101C, A118C, V184C or T205C according to Kabat numbering). In specific examples, a THIOMAB^{™} antibody has a single cysteine mutation in either the heavy or light chain such that each full-length antibody (*i.e.,* an antibody with two heavy chains and two light chains) has two engineered cysteine residues. In specific embodiments, the STEAP1-ADC is a STEAP1 THIOMAB^{™} antibody. In specific embodiments, the STEAP1-ADC is a STEAP1 THIOMAB^{™} antibody with a heavy chain A118C substitution conjugated to MMAE.

The terms "treat" and "treatment" refer to both therapeutic treatment and prophylactic or preventative measures, wherein the object is to prevent or slow down (lessen) an undesired physiological change or disorder, such as the development or spread of cancer. For purposes of this invention, beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i.e.,* slow to some extent and preferably stop) cancer cell infiltration into peripheral organs; inhibit (*i.e.,* slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

The terms "cancer" and "cancerous" refer to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (*e.g.,* epithelial squamous cell cancer), lung cancer including small- cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

A "STEAP1-expressing cancer" is one comprising cells which have STEAP1 protein present at their cell surface. A "STEAP1-expressing cancer" is one which produces sufficient levels of STEAP1 at the surface of cells thereof, such that an anti-STEAP1 antibody can bind thereto and have a therapeutic effect with respect to the cancer.

The "STEAP1-ADC" refers to a STEAP1 antibody that is conjugated to a cytotoxic moiety. In specific embodiments, the STEAP1-ADC is the 15A5 anti-STEAP1 antibody that is conjugated to a cytotoxic moiety using disulfide linkage at intrinsic cysteine residues. In specific embodiments, the STEAP1-ADC is the 15A5 anti-STEAP1 antibody that is conjugated to an MMAE moiety using disulfide linkage at intrinsic cysteine residues. In specific embodiments, the STEAP1-ADC is a THIOMAB^{™} antibody (an anti-STEAP1 THIOMAB^{™} antibody) wherein the THIOMAB^{™} antibody is the 15A5 anti-STEAP1 antibody that is conjugated to a cytotoxic moiety using disulfide linkage at an engineered cysteine residue. In specific embodiments, the anti-STEAP1 THIOMAB^{™} antibody is conjugated to MMAE. In specific embodiments, the anti-STEAP1 THIOMAB^{™} antibody has an engineered cysteine residue at heavy chain A118C.

A cancer which "overexpresses" an antigenic receptor is one which has significantly higher levels of the receptor, such as STEAP1, at the cell surface thereof, compared to a noncancerous cell of the same tissue type. Such overexpression may be caused by gene amplification or by increased transcription or translation. Receptor overexpression may be determined in a diagnostic or prognostic assay by evaluating increased levels of the receptor protein present on the surface of a cell (*e.g.,* via an immunohistochemistry assay; IHC). Alternatively, or additionally, one may measure levels of receptor-encoding nucleic acid in the cell, *e.g.,* via fluorescent in situ hybridization (FISH; see WO 98/45479), southern blotting, or polymerase chain reaction (PCR) techniques, such as real time quantitative PCR (RT-PCR).

An "isolated" antibody is one which has been separated from a component of its natural environment. In some embodiments, an antibody is purified to greater than 95% or 99% purity as determined by, for example, electrophoretic (*e.g.,* SDS-PAGE, isoelectric focusing (IEF), capillary electrophoresis) or chromatographic (*e.g.,* ion exchange or reverse phase HPLC). For review of methods for assessment of antibody purity, see, *e.g.,* Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-STEAP-1 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "package insert" is used to refer to instructions customarily included in commercial packages of therapeutic products, that contain information about the indications, usage, dosage, administration, combination therapy, contraindications and/or warnings concerning the use of such therapeutic products.

### II. METHODS

The methods disclosed herein meet the unmet needs of being able to identify patients who will benefit from STEAP1-ADC therapy and being able to monitor disease progression and STEAP1-ADC therapy efficacy without invasive, surgical biopsies. The methods allow for the detection of patients and selection of patients who are good candidates for STEAP1-ADC therapy and patients who will respond to STEAP1-ADC therapy without invasive and painful biopsies. The methods also allow for the frequent monitoring of STEAP1+ CTCs and STEAP1 expression during treatment with the STEAP1-ADC. Frequent monitoring allows dose adjustments and personalization that surgical biopsies do not allow due to their invasive nature. Accordingly, the methods disclosed herein are particularly important for ADC therapy because ADC therapies are focused on a specific biomarker and it is advantageous to be able to monitor and detect level of that specific biomarker before, during, and after ADC therapy.

In one aspect, the present disclosure provides methods for diagnosing or staging prostate cancer in a test subject using a blood sample from the test subject. In particular, the present disclosure provides method for diagnosing or staging prostate cancer in a test subject by determining whether the circulating tumor cells (CTCs) express one or more prostate-specific markers. In specific embodiments the present disclosure provides methods for diagnosing or staging prostate cancer in a test subject who has or will undergo STEAP1-ADC therapy (*i.e.,* treatment with a STEAP1-ADC).

In the methods provided herein, CTCs are analyzed for expression of one or more prostate specific markers. The detection of prostate specific markers on the CTCs provides further information as to the diagnosis and staging of prostate cancer. The prostate specific marker is STEAP 1.

The CTCs are analyzed for four markers: cytokeratin (CK), CD45, DAPI, and STEAP1. CK is an epithelial marker used to detect CTCs. CD45 is a white blood cell marker, DAPI is a nuclear marker. STEAP1 is a marker for prostate cancer. STEAP1 positive CTCs are identified with a STEAP1+/CK+/DAPI+ signature.

In certain embodiments, the present invention provides methods for diagnosing staging prostate cancer in a test subject, comprising: a) identifying CTCs from a blood sample taken from the test subject wherein the identified CTCs are not separated or enriched from the other components in the blood sample; b) contacting the CTCs with an antibody that specifically binds to STEAP1, an antibody that specifically binds to CD45 and an antibody that specifically binds to cytokeratin; c) further screening the cells for DAPI staining and CK and CD45 expression, d) detecting the DAPI stain and determining whether any of the cancer cells express the prostate-specific marker STEAP 1, CD45, and CK, wherein the anti-STEAP 1 bound antibody is detected with a tyramide signal amplification (TSA) protocol to enhance signal, and wherein the presence of cancer cells that express the prostate-specific marker STEAP1, CK, and stain with DAPI without CD45 expression is predictive of having prostate cancer in the test subject.

The diagnostic and prognostic assays employ an amplification protocol. The amplification protocol is a tyramide signal amplification (TSA) protocol, which can be used, for example, in combination with dyes (e.g., Alexa Fluors) to achieve high-resolution signal amplification in cell applications. TSA is an enzyme-mediated detection method that utilizes the catalytic activity of horseradish peroxidase (HRP) to generate high-density labeling of a target proteins.

The term "cancer cells of epithelial origin" refers to cancer cells that express at least one epithelial marker. The term "marker" as used herein refers to an antigen molecule that is preferentially expressed on a particular type of cells and helps distinguish those cells from other types of cells. For example, an epithelial marker can be an antigen molecule universally expressed on epithelial cells but not normally found on leukocytes. The cancer cells of epithelial origin may also express a tumor marker, for example, an antigen molecule preferentially found on tumor cells but less frequently found on normal cells. In certain embodiments, the cancer cells of epithelial origin comprise CTCs.

In certain embodiments, the cancer cells of epithelial origin express at least one epithelial marker which is also preferentially found in cancer cells. Detection of such an epithelial marker is indicative of a cancer cell of epithelial origin. In certain embodiments, such an epithelial marker is Epithelial Cell Adhesion Molecule (EpCAM).

The cancer cells of epithelial origin are from a blood sample obtained from the test subject. The blood samples can be any sample that is derived from human blood, for example, a plasma sample, a serum sample, whole blood, or blood that has been treated with certain agents such as an anti-coagulant. Blood samples can be obtained directly from the test subjects, or can be obtained from organizations that collect the samples from the test subjects.

In certain embodiments, the cancer cells are identified from the blood sample with a capturing composition. In certain embodiments, the capturing composition comprises a ligand that specifically binds to cancer cells of epithelial origin. In certain embodiments, the ligand is an antibody that specifically binds to an epithelial antigen preferentially expressed on cancer cells. In certain embodiments, the epithelial antigen is EpCAM.

The term "identify" or "identification" as used herein, refers to substantially differentiating the cancer cells of epithelial origin from the rest of the components in the blood sample. For example, the cancer cells of epithelial origin can be bound or captured, while the rest of the components are not bound or captured.

The identified cancer cells are not separated or enriched from the other components in the sample. For example, a blood sample, *e.g*. a serum sample, may be loaded to a slide, identified, and without any separation or enrichment operations, the sample may be further contacted with other reagents.

The application also describes methods in which, cells are enriched in a cell fraction separated from the blood sample. The term "enriched" as used herein refers to the density of the identified cancer cells in the cell fraction is higher than in the blood sample. Any suitable techniques may be used to separate the cell fraction. Techniques commonly employed in the art include, without limitation, gravitational separation, magnetic separation or affinity separation, for example, after the cancer cells form a complex with the capturing composition which allows separation of the cancer cells. For gravitational separation, the capturing composition may be coupled to particles or beads that can be spin down to allow enrichment of the identified cancer cells. For magnetic separation, the capturing composition may be coupled to magnetic particles that can be separated in suitable magnetic fields. For affinity separation, the capturing composition may be immobilized on a device, such as a slide, and allows capture of the identified cells.

In such methods, the cell fraction may be separated under a magnetic field. In such methods, the ligand in the capturing composition may be coupled to a magnetic particle.

Magnetic particles suitable for the methods disclosed herein can be prepared using methods known in the art, see for example, U.S. Patents 5,597,531, 5,698,271, and 6,365,362, and also procedures described in Liberti et al, In Fine Particles Science and Technology, 777-90, E. Pelizzetti (ed.) (1996). Briefly, the magnetic particles comprise a magnetic core (*e.g.* iron oxides) which is coated with polymers or proteins (*e.g.,* bovine serum albumin and casein). The magnetic mass and size of the magnetic particles can be controlled such that the magnetic particles are magnetically responsive yet are substantially invisible to cell analytical techniques such as immunofluorescence detection. The suitable size of the magnetic particles may be less than 200nm, preferably with a suitable size distribution range, for example, within 90-150nm. The magnetic mass of the particles may be between 70-90%.

The magnetic particle may be colloidal. Such colloidal magnetic particles are substantially stable in solution over an extended period of time, and do not tend to aggregate under gravitational force or in the absence of an applied magnetic field. The magnetic particle may be colloidal nanoparticles.

The ligand in the capturing composition can be coupled to the magnetic particles using any suitable methods known in the art. For example, the capturing composition may be direct coupled to a magnetic particle using heterobifunctional linkers, such as succinimidylpropiono-dithiopyridine (SPDP), and sulfosuccinimidil-4-[maleimidomethyl]cyclohexane-1-carboxylate(SMCC)). For another example, the capturing composition comprising a biotinylated antibody may be coupled to a magnetic particle conjugated with streptavidin. The capturing composition and the magnetic particles may also be introduced with other conjugating pairs that can bring about the coupling, for example, avidin-biotin, protein A-Antibody Fc, receptor-ligand, and lectin-carbohydrate.

The capturing composition may be an EpCAM antibody coupled to magnetic colloidal nanoparticles. The CellSearch^{®} System (Veridex, New Jersey) may be used to separate the cell fraction enriched with the identified cells.

The cancer cells of epithelial origin may be contacted with an antibody that specifically binds to a prostate-specific marker. The term "prostate-specific" as used herein indicates that, the marker is preferentially found in prostate tissues, and substantially distinguishes prostate tissues or cells from other tissues or cells. In certain embodiments, the prostate-specific marker is a surface or membrane marker of prostate cells. In certain embodiments, the prostate-specific marker is selected from the group consisting of Six-transmembrane epithelial antigen of the prostate (STEAP-1) (see, *e.g.* Hubert et al., (1999) Proc. Natl. Acad. Sci. USA, 96, 14523-14528), Prostate-specific membrane antigen (PSM) (*see, e.g.,* Israeli, R. S. et al., (1993) Cancer Res. 53, 227-230), Prostate carcinoma tumor antigen (PCTA-1) (*see, e.g.,* Su, Z. Z. et al., (1996) Proc. Natl. Acad. Sci. USA 93, 7252-7257), and Prostate stem cell antigen (PSCA) (*see, e.g.,* Reiter, R. E. et al. (1998) Proc. Natl. Acad. Sci USA 95, 1735-1740). An exemplary human STEAP-1 has an amino acid sequence of SEQ ID NO: 1 disclosed in US 2009/0280056 A1, filed 26 Oct 2007, the entire disclosure of which is.

In certain embodiments, the anti-STEAP-1 antibody binds to STEAP-1 with a K_{D} of ≤ 1000 nM. The anti-STEAP-1 antibody can be a polyclonal antibody or a monoclonal antibody, and can be of any suitable species, such as for example, a sheep antibody, a rabbit antibody, or a murine antibody.

In certain embodiments, the anti-STEAP-1 antibody is a murine monoclonal antibody. In certain embodiments, the anti-STEAP-1 antibody is 15A5, produced by a hybridoma cell having a microorganism deposit number of PTA-12259. In certain embodiments, the anti-STEAP-1 antibody is an antibody which bind to substantially the same epitope to which antibody 15A5 binds.

In certain embodiments, the anti-STEAP-1 antibody is conjugated with a first detectable label. Any suitable detectable labels may be used. In certain embodiments, the detectable label is a fluorescent label, such as for example, fluorophore AF-488, derivatives of cyanine dyes, fluorescein, rhodamine, Texas red, aminomethylcoumarin (AMCA), phycoerythrin, fluorescein isothiocyanante (FITC), among others. Methods of conjugating an antibody with a detectable label are well known in the art, see for example, Hermanson, G. T., Bioconjugate techniques, Academic Press, 2008.

In certain embodiments, the anti-STEAP-1 antibody is not conjugated. The un-conjugated anti-STEAP-1 antibody can be detected with a secondary antibody conjugated with a detectable label (*e.g.* the first detectable label). Such secondary antibody can be any antibody raised in a different species than the anti-STEAP-1 antibody and recognizes the constant region of the anti-STEAP-1 antibody, as is commonly employed in the art.

The cancer cells are identified with reagents that allow detection of CTCs.

The reagents comprise an antibody that specifically binds to STEAP1, an antibody that specifically binds to CD45, and an antibody that specifically binds to cytokeratin. Cytokeratins are a group of proteins typically expressed in epithelial cells, and form keratin-containing filaments in the cytoskeleton of epithelial tissue.

In certain embodiments, the anti-cytokeratin antibody is conjugated with a second detectable label. Any suitable detectable label may be used, for example, a fluorescent label such as phycoerythrin.

The reagents further comprise 4',6-diamidino-2-phenylindole (DAPI) a cell-specific dye that differentiates cells from non-cell components.

The reagents further comprise an anti-CD45 antibody. In certain embodiments, the anti-CD45 antibody,is conjugated with a third detectable label. For example, the ligand can be an anti-CD45 antibody conjugated with allophycocyanin. The staining of the identified cells by an anti-CD45 antibody can be helpful to exclude the leukocytes from the CTCs of epithelial origin.

In case more than one detectable label (including a dye) is used in one testing, it is preferred that the detectable labels are selected such that each label can be independently detected without substantial interference to any other detectable signals present in the sample. For example, the detectable labels (including a dye) may be different fluorescent molecules showing different colors under the detection condition.

The detection can be carried out by any suitable method, for example, those based on immunofluorescent microscopy, flow cytometry, fiber-optic scanning cytometry, or laser scanning cytometry.

In certain embodiments, the cancer cells are visualized under fluorescent microscopy after stained with DAPI and differently labeled antibodies or ligands for cytokeratin, STEAP1 and CD45. The cells positive for DAPI, cytokeratin and STEAP1, but negative for CD45 are classified as cancer cells of epithelial origin that express the prostate-specific marker. Such cells may also be analyzed using flow cytometry, *see,* for example, Cruz, I., et al., Am J Clin Pathol, Vol. 123: 66-74 (2005).

Alternatively, the cancer cells may also be deposited on a surface of a glass slide, and scanned for cells positive for DAPI, cytokeratin and STEAP1, but negative for CD45 (*see,* for example, Marrinucci, D. et al., Human Pathology, Vol. 38, No. 3, 514-519 (2007)). Similarly, the identified cells deposited on a glass slide may also be analyzed using laser-scanning technology (*see,* for example, Pachmann, K. et al., Breast Cancer Research, Vol. 7, No. 6, R975-R979 (2005)).

In certain embodiments, the methods further comprise determining the amount of the cancer cells that express the prostate-specific marker, wherein such amount is predictive of the stage of prostate cancer in the test subject. Assumptions have been made to correlate the size and/or aggressiveness of a tumor with the number of tumor cells in peripheral blood. For example, it is reported that a patient with a 1-mm diameter tumor may have a frequency of tumor cells in peripheral blood of about 6 tumor cells per 100ml blood (*see,* for example, U.S. Patent 6,365,362). Assuming an increase in tumor size may be proportional to this frequency, criteria may be established to indicate the stage of the cancer in the test subject. In certain embodiments, clinical blood samples from patients diagnosed of early stage or metastatic prostate cancer may be used to determine a statistical level of cancer cells in the peripheral blood for those patients, and thereby provides for criteria for future detection and analysis.

In certain embodiments, the methods further comprise determining the expression level of the prostate-specific marker on the cancer cells. Some prostate-specific markers are antigens whose expression level may be up-regulated as a result of tumor growth, metastasis and/or an advanced stage of the cancer. Such prostate-specific markers may include, without limitation, STEAP-1, PSMA, PCTA-1, and PSCA. The expression levels of the prostate-specific markers on the cancer cells may be determined by any suitable methods, for example, by determining the intensity of the fluorescence signal corresponding to the prostate-specific marker.

In certain embodiments, the methods further comprise grading the cancer cells based on their expression level of the prostate-specific marker, and determining the percentage of the cancer cells in each grade. In certain embodiments, cancer cells expressing high level, medium level and low level of the prostate-specific marker are respectively graded. The criteria for "high level", "medium level", and "low level" can be determined, for example, using established cell lines having known expression levels of the marker. For example, the LB50 cell line is known to express a high level of STEAP-1, the LnCAPner cell line is known to express a medium level of STEAP-1, and the PC3 cell line is known to express a low level of STEAP-1. Therefore, cancer cells as detected to have a STEAP-1 expression level comparable to or higher than the LB 50 cell line may be graded as "high level". Similarly, "medium level" may be assigned to cancer cells whose STEAP-1 expression level is comparable to or higher than that of LnCAPner cell line but is lower than that of LB 50 cell line. Those having an STEAP-1 expression level comparable to or lower than that of PC3 cell line may be graded as or "low level."

The number of the cancer cells in each grade may be further determined. In certain embodiments, the percentage of the cancer cells in each grade may be calculated. A higher percentage of cancer cells in the high level grade can be indicative of a more advanced stage of the prostate cancer. Similarly, prostate cancer at an early stage may show lower percentage of cancer cells in the high level grade, and/or higher percentage of cancer cells in low level grade.

In certain embodiments, the methods further comprise calculating a grade score for each grade by multiplying the percentage of the cancer cells in that grade with a unique grade number assigned to that grade based on the expression level of the prostate-specific marker, and summing up all the grade scores to obtain an H score, wherein the H score is indicative of the stage of the prostate cancer in the test subject. For example, a grade number of 3 may be assigned to the high level grade, 2 to the medium level grade, and 1 to the low level grade, which defines the range of the H score within 0 to 300. A higher H score is indicative of more cells in the high level grade, i.e. a more advanced stage of the prostate cancer, and a lower H score indicates more cells in the low level grade, i.e. a relatively early stage of the prostate cancer.

In some embodiments, the methods further comprise determining the presence of a marker and/or frequency of presence of a marker. In some embodiments, the presence of a marker is determined by immunohistochemical ("IHC"), Western blot analysis, immunoprecipitation, molecular binding assays, ELISA, ELIFA, fluorescence activated cell sorting ("FACS"), MassARRAY, proteomics, quantitative blood based assays (as for example Serum ELISA), biochemical enzymatic activity assays, in situ hybridization, Northern analysis, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), as well as any one of the wide variety of assays that can be performed by protein, gene, and/or tissue array analysis. In some embodiments, the presence of a marker is determined by fluorescent in situ hybridization (FISH). In some embodiments, the marker is PTEN. In some embodiments, the CTC is triploid. In some embodiments, the CTC is triploid with PTEN loss. In some embodiments, the CTC is determined to be triploid by CEP10 FISH. In some embodiments, the CTC is determined comprise PTEN loss by PTEN FISH.

In another aspect, the present invention disclosure also provides methods of predicting efficacy of prostate cancer therapy in a test subject, comprising: a) identifying CTCs from a blood sample taken from the test subject wherein the identified CTCs are not separated or enriched from the other components in the blood sample; b) contacting the CTCs-cancer cells of epithelial origin with an antibody that specifically binds to STEAP1, an antibody that specifically binds to CD45, and an antibody that specifically binds to cytokeratin; c) staining the CTCs with DAPI; and d) detecting the DAPI stain and bound antibodies wherein the anti-STEAP1 bound antibody is detected with a tyramide signal amplification (TSA) protocol to enhance signal; and wherein the presence of CTCs that express cytokeratin and STEAP1 and exhibit DAPI staining without CD45 expression is predictive of the efficacy of the prostate cancer therapy in the test subject.

Certain prostate-specific markers may also be therapeutic targets for prostate cancer treatment. Therefore, the expression level of such marker on the CTCs and changes in such level can be predicative of the efficacy of therapies that target such marker.

In certain embodiments, the methods further comprise determining the amount of the cancer cells that express the prostate-specific marker, and/or determining the expression level of a prostate-specific marker on the cancer cells. For example, the expression level of a prostate-specific marker (*e.g.* STEAP-1) on the cancer cells from the baseline pre-treated sample in early phase clinical trials can be correlated with clinical endpoints such as progression free survival, PSA changes, patient-reported bone pain, overall survival, or others, in order to determine whether expression of the prostate-specific marker above a certain threshold is predictive of clinical activity of the prostate cancer therapy (*e.g.* STEAP-1 Antibody-Drug Conjugate (ADC) based therapy). Dynamic changes in expression level of the prostate-specific marker (*e.g.* STEAP-1) in the cancer cells (i.e. down-regulation in post-treatment samples) can also be correlated to clinical outcome measures to determine if such changes are predictive of therapeutic activity. Such methods can be used as a first step in qualifying the assay as a candidate predictive biomarker that could be used to select patients for a prostate cancer therapy (*e.g.* STEAP-1 ADC-based therapy), followed by prospective validation in a confirmatory phase III study.

In another aspect, the present invention also provides methods of monitoring response to a prostate cancer therapy in a test subject, comprising: a) contacting a first group of CTCs with an antibody that specifically binds to STEAP1, an antibody that specifically binds CD45, and an antibody that specifically binds cytokeratin, and staining the cells with DAPI, wherein the first group of cancer cells are from a first blood sample taken from the test subject before treatment with the STEAP 1-ADC; b) determining the number of CTCs in the first group that express STEAP1 and cytokeratin and stain with DAPI; c) contacting a second group of CTCs with an antibody that specifically binds to STEAP1, an antibody that specifically binds CD45, and an antibody that specifically binds cytokeratin, and staining the cells with DAPI, wherein the second group of cancer cells are from a second blood sample taken from the test subject during or after treatment with the STEAP1-ADC d) determining the amount of the cancer cells in the second group that express STEAP1 and cytokeratin and stain with DAPI; and e) comparing the amount of the cancer cells that express STEAP1 and cytokeratin and stain with DAPI as determined in b) with that in step d), wherein a decrease in the amount of the CTCs and/or a decrease in the STEAP1+ CTCs indicates a response to the prostate cancer therapy in the test subject; wherein the anti-STEAP1 bound antibody is detected with a tyramide signal amplification (TSA) protocol to enhance signal; and wherein the CTCs are not separated or enriched from the other components in the blood sample.

### III. Antibodies

The antibodies used herein bind to substantially the same epitope to which antibody 15A5 binds, wherein antibody 15A5 is produced by a hybridoma cell having a microorganism deposit number of PTA-12259.

The antibodies used herein may compete with 15A5 antibody for binding to STEAP-1. Competition assays may be used to identify an antibody that competes with the anti-STEAP-1 antibody 15A5 for binding to STEAP-1.

In an exemplary competition assay, immobilized STEAP-1 is incubated in a solution comprising a first labeled antibody that binds to STEAP-1 (*e.g.,* 15A5) and a second unlabeled antibody that is being tested for its ability to compete with the first antibody for binding to STEAP-1. The second antibody may be present in a hybridoma supernatant. As a control, immobilized STEAP-1 is incubated in a solution comprising the first labeled antibody but not the second unlabeled antibody. After incubation under conditions permissive for binding of the first antibody to STEAP-1, excess unbound antibody is removed, and the amount of label associated with immobilized STEAP-1 is measured. If the amount of label associated with immobilized STEAP-1 is substantially reduced in the test sample relative to the control sample, then that indicates that the second antibody is competing with the first antibody for binding to STEAP-1. *See* Harlow and Lane (1988) Antibodies: A Laboratory Manual ch.14 (Cold Spring Harbor Laboratory, Cold Spring Harbor, NY).

In certain embodiments, the antibodies used herein has a dissociation constant (Kd) to STEAP-1 of ≤ 1000 nM, ≤ 100 nM, ≤ 10 nM, ≤ 1 nM, ≤ 0.1 nM, ≤ 0.01 nM, or ≤ 0.001 nM (*e.g.* 10⁻⁸ M or less, *e.g.* from 10⁻⁸ M to 10⁻¹³ M, *e.g.,* from 10⁻⁹ M to 10⁻¹³ M).

In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (*see, e.g.,* Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (*e.g.,* consistent with assessment of the anti-VEGF antibody, Fab-12, in Presta et al., Cancer Res. 57:4593-4599 (1997)). The Fab of interest is then incubated overnight; however, the incubation may continue for a longer period (*e.g.,* about 65 hours) to ensure that equilibrium is reached. Thereafter, the mixtures are transferred to the capture plate for incubation at room temperature (*e.g.,* for one hour). The solution is then removed and the plate washed eight times with 0.1% polysorbate 20 (TWEEN-20^{®}) in PBS. When the plates have dried, 150 µl/well of scintillant (MICROSCINT-20 ^{™}; Packard) is added, and the plates are counted on a TOPCOUNT ^{™} gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE^{®}-2000 or a BIACORE^{®}-3000 (BIAcore, Inc., Piscataway, NJ) at 25°C with immobilized antigen CM5 chips at ~10 response units (RU). Briefly, carboxymethylated dextran biosensor chips (CM5, BIAcore, Inc.) are activated with *N*-ethyl-*N*'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (~0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{™}) surfactant (PBST) at 25°C at a flow rate of approximately 25 µl/min. Association rates (kₒₙ) and dissociation rates (k_{off}) are calculated using a simple one-to-one Langmuir binding model (BIACORE^{®} Evaluation Software version 3.2) by simultaneously fitting the association and dissociation sensorgrams. The equilibrium dissociation constant (Kd) is calculated as the ratio k_{off}/kₒₙ. *See, e.g.,* Chen et al., J. Mol. Biol. 293:865-881

(1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm bandpass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ^{™} spectrophotometer (ThermoSpectronic) with a stirred cuvette.

Kinetic binding measurements can also be performed on an Octet Red instrument (ForteBio, Menlo Park, CA, USA). For example, all washes, dilutions and measurements are performed in Octet buffer (0.2% dodecylmaltoside, or DDM, - PBS) with the plate shaking at 1000 rpm. Streptavidin biosensors are equilibrated in Octet buffer for 10 min and then loaded with biotinylated STEAP-1 (from viral lysate in 1% DDM, diluted 1:8 in Octet Buffer) for 5 min and washed for 10 min. For the association phase, the ligand-coated streptavidin tips are immersed in anti-STEAP-1 antibody fragments for 10 min (eight serial two-fold dilutions, starting at 500 or 50 nM). Dissociation of the Ab-STEAP-1 complex can be measured in wells containing Octet buffer alone for 600 s. KD, Ka and Kd are determined with Octet evaluation software v6.3 using a 1:1 binding model with global fitting.

In certain embodiments, the antibodies used herein include, without limitation, murine antibodies, sheep antibodies and rabbit antibodies. In certain embodiments, the antibodies are murine monoclonal antibody.

In certain embodiments, the antibodies used herein comprise at least one of the CDR regions of the antibody 15A5. The CDR regions of an antibody can be determined using methods known in the art. Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of ImmunologicalInterest, 5th Ed. Public Health Service, National Institutes of Health, Bethesda, MD (1991).) With the exception of CDR1 in heavy chain variable regions, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, CDR residues and other residues in the variable domain (*e.g*., FR residues) are numbered herein according to Kabat et al., *supra.*

In certain examples the antibodies used herein comprise at least one of the heavy chain variable regions of the antibody 15A5, or at least one of the light chain variable regions of the antibody 15A5.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (*See, e.g.,* Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

Antibody fragments of the antibodies used herein are also encompassed by the present disclosure. In certain examples an antibody used herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, *see, e.g.,* Pluckthün, in The Pharmacology of Monoclonal Antibodies, vol. 113, Rosenburg and Moore eds., (Springer-Verlag, New York), pp. 269-315 (1994); *see also* WO 93/16185; and U.S. Patent Nos. 5,571,894 and 5,587,458. For discussion of Fab and F(ab')₂ fragments comprising salvage receptor binding epitope residues and having increased in vivo half-life, *see* U.S. Patent No. 5,869,046.

Diabodies are antibody fragments with two antigen-binding sites that may be bivalent or bispecific. *See,* for example, EP 404,097; WO 1993/01161; Hudson et al., Nat. Med. 9: 129-134 (2003); and Hollinger et al., Proc. Natl. Acad. Sci. USA 90: 6444-6448 (1993). Triabodies and tetrabodies are also described in Hudson et al., Nat. Med. 9: 129-134 (2003).

Single-domain antibodies are antibody fragments comprising all or a portion of the heavy chain variable domain or all or a portion of the light chain variable domain of an antibody. In certain examples, a single-domain antibody is a human single-domain antibody (Domantis, Inc., Waltham, MA; *see, e.g.,* U.S. Patent No. 6,248,516 B1).

Antibody fragments can be made by various techniques, including but not limited to proteolytic digestion of an intact antibody as well as production by recombinant host cells (*e.g. E. coli* or phage), as described herein.

In certain examples, the antibodies uded herein is antibody 15A5 or its antigen binding fragment.

In certain examples, the antibodies used herein are further conjugated with a detectable label. Suitable labels include, but are not limited to, labels or moieties that are detected directly (such as fluorescent, chromophoric, electron-dense, chemiluminescent, and radioactive labels), as well as moieties, such as enzymes or ligands, that are detected indirectly, *e.g*., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, *e.g*., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, *e.g.*, glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### IV. Nucleic Acids and Host Cells

Antibodies may be produced using recombinant methods and compositions, *e.g.*, as described in U.S. Patent No. 4,816,567. In one example, isolated nucleic acid encoding an anti-STEAP-1 antibody described herein is described. Such nucleic acid may encode an amino acid sequence comprising the VL and/or an amino acid sequence comprising the VH of the antibody (*e.g*., the light and/or heavy chains of the antibody). In a further example, one or more vectors (*e.g*., expression vectors) comprising such nucleic acid are described. In a further example, a host cell comprising such nucleic acid is described. In one such example, a host cell comprises (*e.g*., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one example, the host cell is eukaryotic, *e.g*. a Chinese Hamster Ovary (CHO) cell or lymphoid cell (*e.g.,* Y0, NS0, Sp20 cell). In one example, a method of making an anti-STEAP-1 antibody is described, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-STEAP-1 antibody, nucleic acid encoding an antibody, *e.g*., as described above, is isolated and inserted into one or more vectors for further cloning and/or expression in a host cell. Such nucleic acid may be readily isolated and sequenced using conventional procedures (*e.g*., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, *see, e.g.,* U.S. Patent Nos. 5,648,237, 5,789,199, and 5,840,523. (*See also* Charlton, Methods in Molecular Biology, Vol. 248 (B.K.C. Lo, ed., Humana Press, Totowa, NJ, 2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. *See* Gerngross, Nat. Biotech. 22: 1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See, e.g.,* US Patent Nos. 5,959,177, 6,040,498, 6,420,548, 7,125,978, and 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36:59 (1977)); baby hamster kidney cells (BHK); mouse sertoli cells (TM4 cells as described, *e.g.,* in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.,* in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, *see, e.g.,* Yazaki and Wu, Methods in Molecular Biology, Vol. 248 (B.K.C. Lo, ed., Humana Press, Totowa, NJ), pp. 255-268 (2003).

A hybridoma cell line is also described herein, having a microorganism deposit number of PTA-12259. The hybridoma cell line produces the antibody 15A5.

### V. Use of the Antibodies

The antibodies used herein can be used in the manufacture of a diagnostic reagent for prostate cancer. The antibodies may be further conjugated with a detectable label suitable for the diagnostic purpose, and may be presented in a suitable form, such as in lyophilized powers or in suitable solution form.

### VI. Test Kits

The application describes test kits containing compositions useful for the diagnosis or prognosis of prostate cancer

The present disclosure further describes test kits for detecting presence of prostate cancer cells expressing STEAP-1 in a blood sample, comprising an antibody that specifically binds to STEAP-1.

In certain examples, the antibody is conjugated with a first detectable label. Any suitable detectable label may be used, such as fluorescent label.

In certain examples, the antibody is an anti-STEAP-1 antibody. In certain examples, the antibody is antibody 15A5.

In certain examples, the test kits further comprise one or more compositions selected from the group consisting of: magnetic particles coupled to a first ligand that specifically binds to cancer cells of epithelial origin, a second ligand that specifically binds to an epithelial marker; a third ligand specifically binds to a leukocyte marker, and a dye that differentiates cells from non-cell components.

In certain examples, the second ligand is conjugated to a second detectable label, and/or the third ligand is conjugated to a third detectable label. In certain examples, when the test kits comprises more than one detectable label (including a dye), it is preferred that the detectable labels (including a dye) are selected such that each label can be independently detected without substantial interference to any other detectable signals present in the sample.

In certain examples, the first ligand, the second ligand and/or the third ligand comprises In certain examples, the first ligand comprises an anti-EpCAM antibody. In certain examples, the second ligand comprises an anti-keratin antibody. In certain examples, the third ligand comprises an anti-CD45 antibody.

The test kits can further comprise a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for diagnosing the condition. At least one reagent in the composition is an antibody of the invention. The label or package insert indicates that the composition is used for in vitro diagnosis of the condition of choice.

### VII. EXAMPLES

The following are examples of methods and compositions of the invention. It is understood that various other embodiments may be practiced, given the general description provided above.

### Example 1. Immunofluoresence-Based Detection of STEAP1 Expression Levels on CTCs

As shown in this Example, an immunofluorescence-based assay was capable of demonstrating robust detection of STEAP1 on the surface of CTCs, and a reduction of the total number of CTCs as well as STEAP 1+ CTCs after treatment.

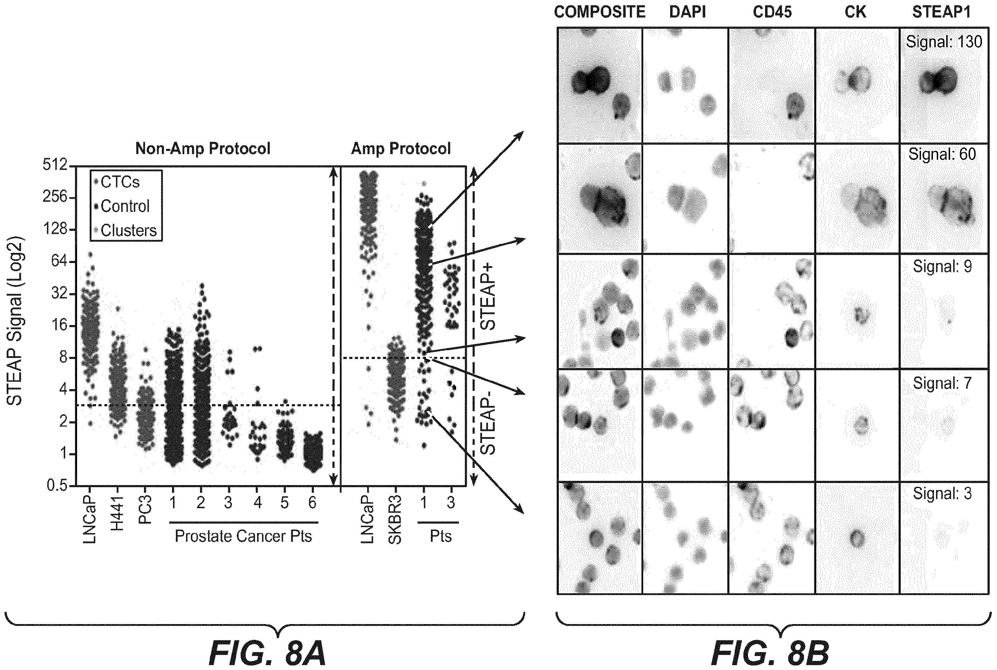

Samples were processed and analyzed using a four channel Epic CTC platform as described herein. Blood samples were collected from 42 patients with metastatic castration-resistant prostate cancer (mCRPC) before and after treatment with the STEAP1-ADC. Upon receipt of the patient blood samples at Epic Sciences, the whole blood was lysed and the nucleated cells (approx. 3×10⁶ per slide) were deposited onto microscope slides and frozen at -80°C until analysis. Upon thawing, the slides were stained with a cocktail including anti-cytokeratin antibody, anti-CD45 antibody, DAPI stain and 15A5 anti-STEAP 1 antibody for the evaluation of this protein biomarker expression on CTCs. The anti-cytokeratin antibody, anti-CD45 antibody, and the DAPI stain were used to identify CTCs. The anti-STEAPl 15A5 antibody was used to detect STEAP1 expressing CTCs. The assays were developed to measure STEAP1 on the surface of CTCs using a traditional protocol without amplification ("Non-Amp") and a signal amplification protocol ("Amp"). The Amp protocol has a signal-to-noise ratio and dynamic range that is 4x and 5x higher than Non-Amp, respectively (Figure 8A). Stained slides were subsequently scanned and the resulting images were analyzed using a multiparametric digital pathology algorithm to detect CTC candidates and quantitate STEAP1 levels. Representative 10X immunofluorescence images for the DAPI (blue), cytokeratin (red), CD45 (green) (Marrinucci et al. J Oncol. 2010: 861341(2010); Cho et al., Phys Biol. 9: 016001 (2012); Marrinucci et al., Phys Biol. 9: 016003 (2012)) and STEAP1 (white) channels are shown for CTC subtypes and the surrounding white blood cells (WBCs), with the 4-channel merge to the far left of each image in Figure 8B.

A reduction in the total number of CTCs/ml, as well as a reduction in the number of STEAP1+ and an increase in STEAP1- CTCs was observed after treatment with the STEAP1-ADC. In particular, STEAP1 expression levels on CTCs were readily detectable in 18/42 samples (43%, 30%-57%), with a range from 1-56% of STEAP1+ CTCs per case. After treatment, a decrease in the fraction of patients with STEAP1-positive and an increase in the STEAP1- CTCs were observed compared to baseline.

### Example 2. Use of CTC assay to evaluate ADC prognosis and efficacy

Sixty patients with progressive mCRPC received doses ranging from 0.3 to 2.8 mg/kg of the STEAP1-ADC once every three weeks. ADC activity was measured by PSA declines and time on study for >6 months. At doses of ≥ 2 mg/kg, PSA declines of ≥ 50% were observed in 10/45 (22% ± 12.1) patients. ADC activity was most pronounced in patients with IHC 3+ tumors. At ≥ 2 mg/kg, 20/45 patients (44% ± 14.5) had unfavorable CTC counts of ≥5 per 7.5mL at baseline and were considered evaluable. After the first treatment cycle, CTC conversions from unfavorable to favorable counts were seen in 11/20 patients (55% ± 21.8) and were significantly correlated with PSA declines (Spearman rank r = 0.74). CTCs showed readily detectable STEAP1+ signal in EpCAM+/CD45-events in 14/29 samples (49% ± 31-66%) by FACS. STEAP1+ events showed EpCAM+/CD45-characteristics in a range from 0 -74%, and FACS sorted EpCAM+/CD45- events demonstrated STEAP1+ RT-PCR expression, along putative markers of prostate cancer AR and KLK3. By EPIC, STEAP1 was detected in 18/42 samples (43% ± 14.98), with a range from 1-56% of STEAP1+ CTCs. After treatment, a decrease in the fraction of patients with STEAP1+ and an increase in the STEAP1= CTCs was observed compared to baseline. 17 patients enrolled on STEAP1 PET study. Of the 14 patients treated with the STEAP1-ADC, 4/14 (29% ± 12-55%) patients had ≥ 50% decline in PSA. Time on treatment correlated with bone metastasis SUVmax (r = 0.63). All 8 PET-guided biopsies of high SUV metastasis were confirmed STEAP1 IHC 2+/3+.

Accordingly, patients with 2+/3+ tumors can be selected based on STEAP1/EpCAM/CD45 expression patterns (described above) or STEAP1/CD45/DAPI expression patterns (described in Example 1) in tissue or blood as predictive biomarkers for STEAP1-ADC sensitivity, and to assess the value of STEAP1 expression on CTCs as a surrogate endpoint for selecting patients who are most likely to benefit from treatment with the STEAP 1-ADC.

### Example 3. The signal amplification protocol increases the dynamic range of the STEAP1 CTC assay.

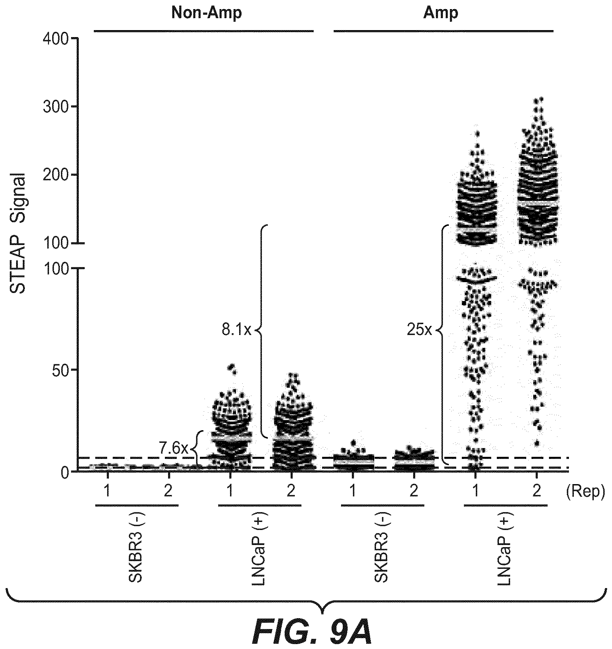

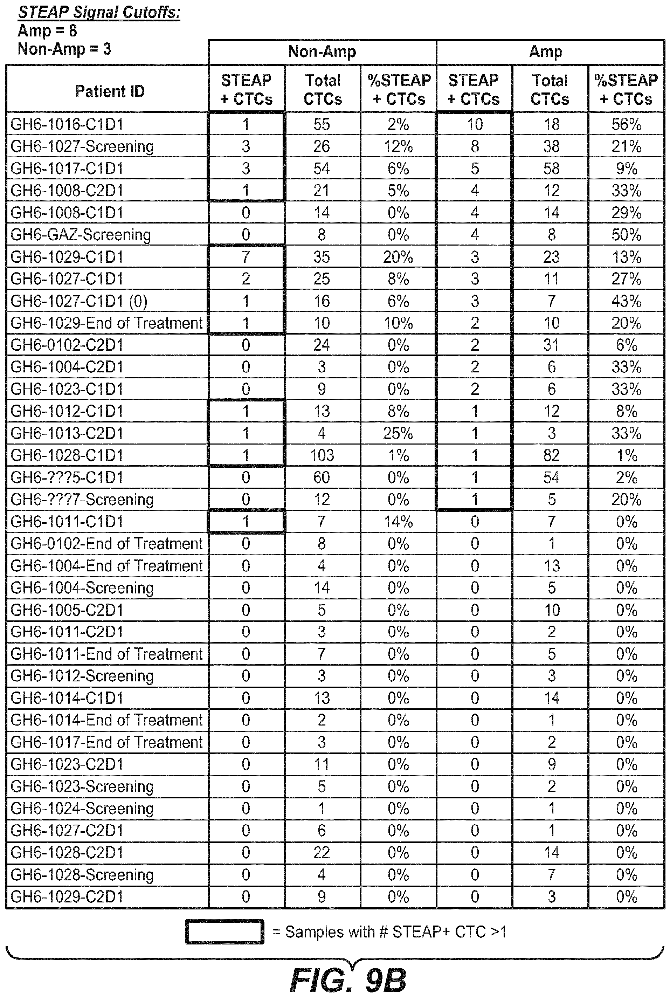

STEAP1 signal was measured in SKBR3 (STEAP1 negative control) and LnCaP (STEAP1 positive control) prostate cancer cell lines in the presence and absence of the amplification protocol (**FIG. 9**). The cut offs for STEAP+ samples was a signal >3 using the non-amp protocol and a signal >8 using the amplification protocol. The non-amp protocol showed a 7.6-fold signal increase in LnCaP (positive control) over SKBR3 (negative control) cell lines. The amp protocol showed a 25-fold signal increase in LnCaP (positive control) over SKBR3 (negative control) cell lines (**FIG. 9A**). STEAP1 signal amplification resulted in a 8.1-fold increase in mean STEAP1 signal for the LnCaP (positive control) cell line while only increasing the background (*i.e.,* the SKBR3 negative control signal) by 2.4-fold (**FIG. 9A**).

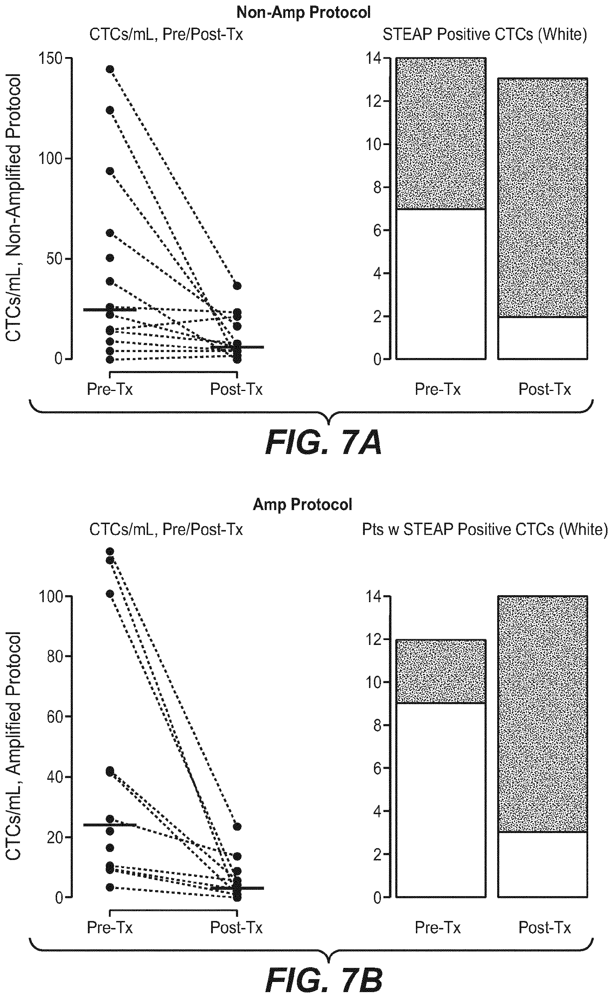

The STEAP1 amp protocol also revealed STEAP1 positive patients, thereby providing a prognostic and diagnostic test which is more sensitive than existing tests (**FIG. 9B**). Out of 36 samples, 12/36 (33%) were STEAP1+ using the non-amp protocol whereas 18/36 (50%) were STEAP1 + using the amp protocol (wherein the cutoff = 1 for STEAP1 + CTCs) (**FIG. 9B**). As shown in Figure 9B, 7 patient samples that would have been classified as STEAP1- using the old non-amp protocol were classified as STEAP1+ using the amp assay.

For STEAP1+ samples, the amp assay was able to detect more STEAP1+ CTCs (*i.e.* samples) as compared to the STEAP1 non-amp assay (**FIG. 9B**). Accordingly, the STEAP1 amp protocol allows identification of patients who will benefit from STEAP 1-ADC treatment who would not have been identified using the traditional non-amp protocol (*e.g.,* patients with low numbers of STEAP1+ CTCs who would not be identified using the traditional non-amp protocol).

### Example 4. STEAP1-ADC treatment results in a decrease in both total CTCs/mL and STEAP1+ CTC counts.

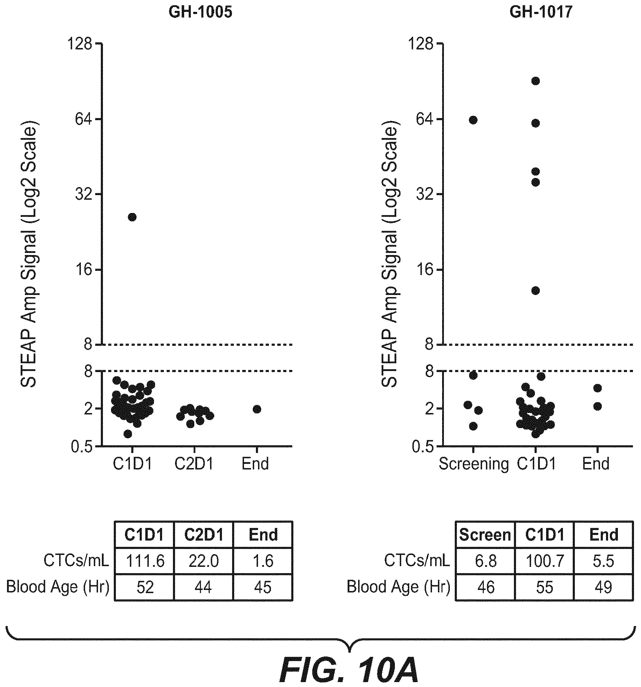

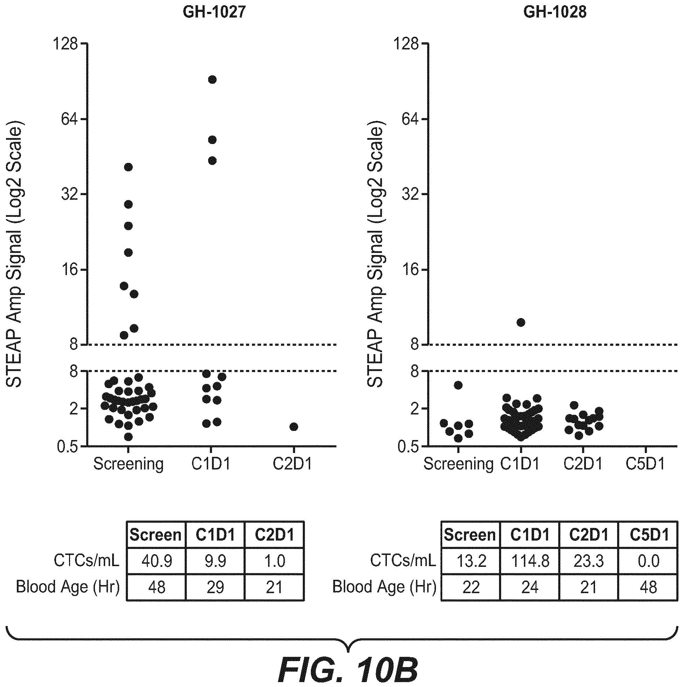

Blood samples were taken from patients before, during, and after treatment with the STEAP1-ADC. As can be seen in Figure 10, as treatment progressed, the number of STEAP1+ CTCs decreased.

## Claims

1. A method for diagnosing prostate cancer in a test subject using circulating tumor cells (CTCs), comprising:
a) identifying CTCs from a blood sample taken from the test subject wherein the identified CTCs are not separated or enriched from the other components in the blood sample;
b) contacting the CTCs with an antibody that specifically binds to STEAP1, an antibody that specifically binds to CD45, and an antibody that specifically binds to cytokeratin;
c) staining the CTCs with DAPI; and
d) detecting the DAPI stain and bound antibodies
wherein the anti-STEAP1 bound antibody is detected with a tyramide signal amplification (TSA) protocol to enhance signal; and
wherein the presence of CTCs that express cytokeratin and the prostate-specific marker STEAP1 and exhibit DAPI staining without CD45 expression is predictive of the test subject having prostate cancer.

2. The method of claim 1, wherein anti-STEAP1 antibody is the 15A5 antibody, produced by a hybridoma cell having a microorganism deposit number of PTA-12259.

3. The method of claim 1 or 2, further comprising determining the expression level of STEAP1 on the cancer cells.

4. The method of any one of claims 1-3, further comprising grading the cancer cells based on their expression level of STEAP1, and determining the percentage of the cancer cells in each grade.

5. The method of any one of claims 1-4, further comprising calculating a grade score for each grade by multiplying the percentage of the cancer cells in said grade with a unique grade number representative of the expression level of the prostate-specific marker in said grade, and summing up all the grade scores to obtain an H score, wherein the H score is indicative of the stage of the prostate cancer in the test subject.

6. The method of any one of claims 1-5, wherein the anti-STEAP-1 antibody binds to STEAP- 1 with a K_{D} of ≤ 1000 nM.

7. The method of any one of claims 1-6, wherein the anti-STEAP-1 antibody, the anti-CD45 antibody, and/or the anti-cytokeratin antibody are conjugated with detectable labels.

8. A method of predicting efficacy of STEAP1-antibody drug conjugate (ADC) therapy in a test subject suffering from prostate cancer, comprising:
a) identifying CTCs from a blood sample taken from the test subject wherein the identified CTCs are not separated or enriched from the other components in the blood sample;
b) contacting the CTCs with an antibody that specifically binds to STEAP1, an antibody that specifically binds to CD45, and an antibody that specifically binds to cytokeratin;
c) staining the CTCs with DAPI; and
d) detecting the DAPI stain and bound antibodies
wherein the anti-STEAP1 bound antibody is detected with a tyramide signal amplification (TSA) protocol to enhance signal; and
wherein the presence of CTCs that express cytokeratin and STEAP1 and exhibit DAPI staining without CD45 expression is predictive of efficacy of the STEAP1-ADC.

9. A method of monitoring response to STEAP1-antibody drug conjugate (ADC) therapy in a test subject suffering from prostate cancer using a tyramide signal amplification (TSA) protocol to enhance signal, comprising:
a) contacting a first group of circulating tumor cells (CTCs) with an antibody that specifically binds to STEAP1, an antibody that specifically binds CD45, and an antibody that specifically binds cytokeratin, and staining the cells with DAPI, wherein the first group of cancer cells are from a first blood sample taken from the test subject before treatment with the STEAP1- ADC;
b) determining the number of CTCs in the first group that express STEAP1 and cytokeratin and stain with DAPI;
c) contacting a second group of CTCs with an antibody that specifically binds to STEAP1, an antibody that specifically binds CD45, and an antibody that specifically binds cytokeratin, and staining the cells with DAPI, wherein the second group of cancer cells are from a second blood sample taken from the test subject during or after treatment with the STEAP 1-ADC;
d) determining the amount of the cancer cells in the second group that express STEAP1 and cytokeratin and stain with DAPI; and
e) comparing the amount of the CTCs that express STEAP1 and cytokeratin and stain with DAPI as determined in b) with that in d),
wherein a decrease in the amount of the CTCs and/or a decrease in the STEAP1+ CTCs indicates a response to the prostate cancer therapy in the test subject;
wherein the anti-STEAP1 bound antibody is detected with a tyramide signal amplification (TSA) protocol to enhance signal; and
wherein the CTCs are not separated or enriched from the other components in the blood sample.

10. The method of claim 8 or 9, wherein the STEAP1-ADC comprises an anti-STEAP1 antibody covalently attached to a cytotoxic agent.

11. The method of claim 10, wherein the cytotoxic agent is selected from a toxin, a chemotherapeutic agent, a drug moiety, monomethylauristatin E (MMAE), an antibiotic, a radioactive isotope, and a nucleolytic enzyme.

12. The method of any one of claims 1-11, wherein the DAPI, STEAP1, CD45, and cytokeratin signals are detected using FACS.

13. The method of any one of claims 1-11, wherein the DAPI, STEAP1, CD45, and cytokeratin signals are detected using immunohistochemistry.

## Patentansprüche

1. Verfahren zur Diagnose von Prostatakrebs in einem Testindividuum unter Verwendung zirkulierender Tumorzellen (CTC), das Folgendes umfasst:
a) das Identifizieren von CTC aus einer Blutprobe, die dem Testindividuum entnommen wurde, wobei die identifizierten CTC nicht von anderen Komponenten in der Blutprobe getrennt oder denen gegenüber angereichert werden,
b) das Kontaktieren der CTC mit einem Antikörper, der spezifisch an STEAP1 bindet, einem Antikörper, der spezifisch an CD45 bindet, und einem Antikörper, der spezifisch an Zytokeratin bindet,
c) das Färben der CTC mit DAPI und
d) das Detektieren der DAPI-Färbung und der gebundenen Antikörper;
wobei der gebundene Anti-STEAP1-Antikörper nach einer Tyramidsignalverstärkungs-(TSA-) Vorschrift zum Verbessern des Signals detektiert wird; und
wobei die Gegenwart von CTC, die Zytokeratin und den prostataspezifischen Marker STEAP1 exprimieren und eine DAPI-Färbung ohne CD45-Expression aufweisen, darauf hindeutet, dass das Testindividuum an Prostatakrebs leidet.

2. Verfahren nach Anspruch 1, wobei der Anti-STEAP1-Antikörper der 15A5-Antikörper ist, der von einer Hybridomzelle mit der Mikroorganismus-Hinterlegungsnummer PTA-12259 produziert wird.

3. Verfahren nach Anspruch 1 oder 2, das weiters das Bestimmen des Expressionsausmaßes von STEAP1 auf den Krebszellen umfasst.

4. Verfahren nach einem der Ansprüche 1 bis 3, das weiters das Einstufen der Krebszellen basierend auf ihrem Expressionsausmaß von STEAP1 in Stadien und das Bestimmen des Prozentanteils der Krebszellen in jedem Stadium umfasst.

5. Verfahren nach einem der Ansprüche 1 bis 4, das weiters das Berechnen einer Stadiumbewertung für jedes Stadium durch Multiplizieren des Prozentsatzes der Krebszellen in dem Stadium mit einer eindeutigen Stadiumzahl des Expressionsausmaßes des prostataspezifischen Markers in dem Stadium und das Addieren aller Stadiumbewertungen umfasst, um eine H-Bewertung zu erhalten, wobei die H-Bewertung das Stadium des Prostatakrebses in dem Testindividuum angibt.

6. Verfahren nach einem der Ansprüche 1 bis 5, wobei der Anti-STEAP1-Antikörper mit einer K_{D} von ≤ 1.000 nM an STEAP1 bindet.

7. Verfahren nach einem der Ansprüche 1 bis 6, wobei der Anti-STEAP1-Antikörper, der Anti-CD45-Antikörper und/oder der Anti-Zytokeratin-Antikörper an detektierbare Markierungen konjugiert ist/sind.

8. Verfahren zum Vorhersagen der Wirksamkeit einer STEAP1-Antikörper-Arzneimittel-Konjugat- (ADC-) Therapie in einem Testindividuum, das an Prostatakrebs leidet, das Folgendes umfasst:
a) das Identifizieren von CTC aus einer Blutprobe, die dem Testindividuum entnommen wurde, wobei die identifizierten CTC nicht von anderen Komponenten in der Blutprobe getrennt oder denen gegenüber angereichert werden,
b) das Kontaktieren der CTC mit einem Antikörper, der spezifisch an STEAP1 bindet, einem Antikörper, der spezifisch an CD45 bindet, und einem Antikörper, der spezifisch an Zytokeratin bindet,
c) das Färben der CTC mit DAPI und
d) das Detektieren der DAPI-Färbung und der gebundenen Antikörper;
wobei der gebundene Anti-STEAP1-Antikörper nach einer Tyramidsignalverstärkungs-(TSA-) Vorschrift zum Verbessern des Signals detektiert wird; und
wobei die Gegenwart von CTC, die Zytokeratin und STEAP1 exprimieren und eine DAPI-Färbung ohne CD45-Expression aufweisen, auf die Wirksamkeit des STEAP1-ADC hindeutet.

9. Verfahren zum Kontrollieren der Reaktion auf eine STEAP1-Antikörper-Arzneimittel-Konjugat- (ADC-) Therapie in einem Testindividuum, das an Prostatakrebs leidet, unter Anwendung einer Tyramidsignalverstärkungs- (TSA-) Vorschrift zum Verstärken des Signals, das Folgendes umfasst:
a) das Kontaktieren der ersten Gruppe von zirkulierenden Tumorzellen (CTC) mit einem Antikörper, der spezifisch an STEAP1 bindet, einem Antikörper, der spezifisch an CD45 bindet, und einem Antikörper, der spezifisch an Zytokeratin bindet, und das Färben der Zellen mit DAPI, wobei die erste Gruppe von Krebszellen aus einer ersten Blutprobe stammt, die dem Testindividuum vor der Behandlung mit dem STEAP1-ADC entnommen wurde,
b) das Bestimmen der Anzahl an CTC in der ersten Gruppe, die STEAP1 und Zytokeratin exprimieren, und das Färben mit DAPI,
c) das Kontaktieren einer zweiten Gruppe von CTC mit einem Antikörper, der spezifisch an STEAP1 bindet, einem Antikörper, der spezifisch an CD45 bindet, und einem Antikörper, der spezifisch an Zytokeratin bindet, und das Färben der Zellen mit DAPI, wobei die zweite Gruppe von Krebszellen aus einer zweiten Blutprobe stammt, die dem Testindividuum während oder nach der Behandlung mit dem STEAP1-ADC entnommen wurde,
d) das Bestimmen der Anzahl an Krebszellen in der zweiten Gruppe, die STEAP1 und Zytokeratin exprimieren, und das Färben mit DAPI und
e) das Vergleichen der unter b) bestimmten Anzahl an CTC, die STEAP1 und Zytokeratin exprimieren, und mit DAPI gefärbt wurden, mit der unter d) bestmmten;
wobei eine Verringerung der Anzahl an CTC und/oder eine Verringerung der STEAP1+CTC eine Reaktion auf die Prostatakrebstherapie in dem Testindividuum anzeigt;
wobei der gebundene Anti-STEAP1-Antikörper nach einer Tyramidsignalverstärkungs-(TSA-) Vorschrift zur Verbesserung des Signals detektiert wird; und
wobei die CTC nicht von den anderen Komponenten in der Blutprobe getrennt oder denen gegenüber angereichert werden.

10. Verfahren nach Anspruch 8 oder 9, wobei das STEAP1-ADC einen Anti-STEAP1-Antikörper umfasst, der kovalent an ein zytotoxisches Mittel gebunden ist.

11. Verfahren nach Anspruch 10, wobei das zytotoxische Mittel aus einem Toxin, einem Chemotherapeutikum, einer Arzneimittelgruppierung, Monomethylauristatin E (MMAE), einem Antibiotikum, einem radioaktiven Isotop und einem nucleolytischen Enzym ausgewählt ist.

12. Verfahren nach einem der Ansprüche 1 bis 11, wobei die DAPI-, STEAP1-, CD45-und Zytokeratin-Signale unter Anwendung von FACS detektiert werden.

13. Verfahren nach einem der Ansprüche 1 bis 11, wobei die DAPI, STEAP1-, CD45- und Zytokeratin-Signale unter Anwendung von Immunhistochemie detektiert werden.

## Revendications

1. Procédé pour diagnostiquer le cancer de la prostate chez un sujet de test en utilisant des cellules tumorales circulantes (CTC), comprenant les étapes consistant à :
a) identifier des CTC à partir d'un échantillon de sang prélevé chez le sujet de test, dans lequel les CTC identifié »s ne sont pas séparées ou enrichies des autres composants de l'échantillon de sang ;
b) mettre en contact les CTC avec un anticorps qui se lie spécifiquement à SIEAP1, un anticorps qui se lie spécifiquement à CD45 et un anticorps qui se lie spécifiquement à la cytokératine ;
c) colorer les CTC avec du DAPI ; et
d) détecter la coloration de DAPI et les anticorps liés
dans lequel l'anticorps lié à anti-STEAPl est détecté en utilisant un protocole d'amplification de signal tyramide (TSA) pour améliorer le signal ; et
dans lequel la présence de CTC qui expriment la cytokératine et le marqueur spécifique de la prostate STEAP1 et présentent une coloration de DAPI sans expression de CD45 est prédictive du sujet de test présentant un cancer de la prostate.

2. Procédé selon la revendication 1, dans lequel l'anticorps anti-STEAPl est l'anticorps 15A5, produit par une cellule d'hybridome présentant un numéro de dépôt de microorganisme de PTA-12259.

3. Procédé selon la revendication 1 ou 2, comprenant en outre la détermination du niveau d'expression de STEAP1 sur les cellules cancéreuses.

4. Procédé selon l'une quelconque des revendications 1 à 3, comprenant en outre la gradation des cellules cancéreuses sur la base de leur niveau d'expression de STEAP1, et la détermination du pourcentage des cellules cancéreuses dans chaque grade.

5. Procédé selon l'une quelconque des revendications 1 à 4, comprenant en outre le calcul d'un score de grade pour chaque grade en multipliant le pourcentage des cellules cancéreuses dans ledit grade par un numéro de grade unique représentatif du niveau d'expression du marqueur spécifique de la prostate dans ledit grade et en additionnant tous les scores de grade pour obtenir un score H, dans lequel le score H est indicatif du stade du cancer de la prostate chez le sujet de test.

6. Procédé selon l'une quelconque des revendications 1 à 5, dans lequel l'anticorps anti-STEAP-1 se lie à STEAP-1 avec une K_{D} ≤ 1 000 nM.

7. Procédé selon l'une quelconque des revendications 1 à 6, dans lequel l'anticorps anti-STEAP-1, l'anticorps anti-CD45 et/ou l'anticorps anti-cytokératine sont conjugués à des marqueurs détectables.

8. Procédé de prédiction de l'efficacité d'un traitement par conjugué médicament-anticorps STEAP1 (ADC) chez un sujet de test souffrant d'un cancer de la prostate, comprenant les étapes consistant à :
a) identifier des CTC à partir d'un échantillon de sang prélevé chez le sujet de test, dans lequel les CTC identifié »s ne sont pas séparées ou enrichies des autres composants de l'échantillon de sang ;
b) mettre en contact les CTC avec un anticorps qui se lie spécifiquement à STEAP1, un anticorps qui se lie spécifiquement à CD45 et un anticorps qui se lie spécifiquement à la cytokératine ;
c) colorer les CTC avec du DAPI ; et
d) détecter la coloration de DAPI et les anticorps liés
dans lequel l'anticorps lié à anti-STEAPl est détecté en utilisant un protocole d'amplification de signal tyramide (TSA) pour améliorer le signal ; et
dans lequel la présence de CTC qui expriment la cytokératine et STEAP1 et présentent une coloration de DAPI sans expression de CD45 est prédictive de l'efficacité du SIEAP1-ADC.

9. Procédé de surveillance de la réponse à un traitement par conjugué de médicament anticorps STEAP1 (ADC) chez un sujet de test souffrant d'un cancer de la prostate en utilisant un protocole d'amplification de signal tyramide (TSA) pour améliorer le signal, comprenant les étapes consistant à :
a) mettre en contact un premier groupe de cellules tumorales circulantes (CTC) avec un anticorps qui se lie spécifiquement à STEAP1, un anticorps qui se lie spécifiquement à CD45 et un anticorps qui se lie spécifiquement à la cytokératine, et colorer les cellules avec du DAPI, dans lequel le premier groupe de cellules cancéreuses provient d'un premier échantillon de sang prélevé chez le sujet de test avant le traitement avec le STEAP1-ADC ;
b) déterminer le nombre de CTC dans le premier groupe qui expriment STEAP1 et la cytokératine, et colorer avec du DAPI ;
c) mettre en contact un second groupe de CTC avec un anticorps qui se lie spécifiquement à STEAP1, un anticorps qui se lie spécifiquement à CD45 et un anticorps qui se lie spécifiquement à la cytokératine, et colorer les cellules avec du DAPI, dans lequel le second groupe de cellules cancéreuses provient d'un second échantillon de sang prélevé chez le sujet de test pendant ou après le traitement avec le STEAP1-ADC ;
d) déterminer la quantité de cellules cancéreuses dans le second groupe qui expriment STEAP1 et la cytokératine, et colorer avec du DAPI ; et
e) comparer la quantité des CTC qui expriment STEAP1 et la cytokératine et colorer avec du DAPI, comme déterminé en b), avec celle en d),
dans lequel une diminution de la quantité des CTC et/ou une diminution des CTC + STEAP1 indique une réponse au traitement du cancer de la prostate chez le sujet de test ;
dans lequel l'anticorps lié à anti-STEAPl est détecté en utilisant un protocole d'amplification de signal tyramide (TSA) pour améliorer le signal ; et
dans lequel les CTC ne sont pas séparées ou enrichies des autres composants de l'échantillon de sang.

10. Procédé selon la revendication 8 ou 9, dans lequel le STEAP1-ADC comprend un anticorps anti-STEAPl fixé de manière covalente à un agent cytotoxique.

11. Procédé selon la revendication 10, dans lequel l'agent cytotoxique est choisi parmi une toxine, un agent chimiothérapeutique, une fraction de médicament, la monométhylauristatine E (MMAE), un antibiotique, un isotope radioactif et une enzyme nucléolytique.

12. Procédé selon l'une quelconque des revendications 1 à 11, dans lequel les signaux de DAPI, STEAP1, CD45 et cytokératine sont détectés en utilisant des FACS.

13. Procédé selon l'une quelconque des revendications 1 à 11, dans lequel les signaux de DAPI, STEAP1, CD45 et cytokératine sont détectés en utilisant une immunohistochimie.
